# EUROPEAN PATENT APPLICATION

(11) **EP 3 922 721 A1**
(43) Date of publication of application: **15.12.2021**
(21) Application number: 20769915.8
(22) Date of filing: 24.02.2020
(51) Int. Cl.: C12N 15/113, C12N 15/62, C07K 16/28, C12N 5/10, C12N 15/85, A61K 48/00, A61P 35/00

(54) **HYPOXIA-REGULATED PROMOTER AND APPLICATION THEREOF**

(30) Priority: 12.03.2019 CN 201910184426; 05.12.2019 CN 201911236679; 05.12.2019 CN 201911236670
(71) Applicant: Chongqing Precision Biotech Company Limited, Chongqing 400000 (CN)
(72) Inventor: ZHANG, Wei, Chongqing 400000 (CN); CHEN, Jun, Chongqing 400000 (CN); QI, Yanan, Chongqing 400000 (CN); XU, Yanmin, Chongqing 400000 (CN); ZHAO, WenXu, Chongqing 400000 (CN); HUANG, Xia, Chongqing 400000 (CN); ZHAO, YongChun, Chongqing 400000 (CN)
(74) Representative: Canzler & Bergmeier Patentanwälte Partnerschaft mbB
(86) International application number: PCT/CN2020/076429
(87) International publication number: WO 2020/181983

(57) **Abstract**

A CAR structure containing hypoxia-regulated promoter, an expression vector, a CAR-T cell and an application thereof, as well as a method for enhancing the ability of the CAR-T cell in secreting IFN-γ and/or IL-2 factors under an anaerobic environment and a method for improving the killing ability of the CAR-T cell under the anaerobic environment. The CAR structure contains the hypoxia-controllable promoter, and the hypoxia-controllable promoter is composed of a Hifla regulatory element and a mini-promoter. The number of repetitions of the Hifla regulatory element is 3-5. The CAR structure may be used for targeted therapy of tumors.

Sequence Listing

<110> CHONGQING PRECISION BIOTECH COMPANY LIMITED

<120> HYPOXIA-REGULATED PROMOTER AND APPLICATION THEREOF

<130> CPB-09962-19

<150> 201910184426.X; 201911236679.3; 201911236670.2
<151> 2019-03-12; 2019-12-05; 2019-12-05

<160> 58

<170> PatentIn Version 3.5

<210> 1
<211> 119
<212> DNA
<213> Artificial sequence

<400>1
cccacagtgc atacgtgggc tccaacaggt cctcttgtcg agccacagtg catacgtggg
60
ctccaacagg tcctcttgtc gagccacagt gcatacgtgg gctccaacag gtcctcttg
119
<210> 2
<211> 139
<212> DNA
<213> Artificial sequence
<400> 2
gtcgacgata tcccacagtg catacgtggg ctccaacagg tcctcttgtc gagccacagt
60
gcatacgtgg gctccaacag gtcctcttgt cgagccacag tgcatacgtg ggctccaaca
120
ggtcctcttg tcgagatct                                              139
<210> 3
<211> 192
<212> DNA
<213> Artificial sequence
<400> 3
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagatctggt aggcgtgtac ggtgggaggt ctatataagc agagctcgtt tagtgaaccg
180
tcagatcact ag
<210> 4
<211> 158
<212> DNA
<213> Artificial sequence
<400> 4
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagccacagt gcatacgtgg gctccaacag gtcctctt
158

<210> 5
<211> 179
<212> DNA
<213> Artificial sequence
<400> 5
gtcgacgata tcccacagtg catacgtggg ctccaacagg tcctcttgtc gagccacagt
60
gcatacgtgg gctccaacag gtcctcttgt cgagccacag tgcatacgtg ggctccaaca
120
ggtcctcttg tcgagccaca gtgcatacgt gggctccaac aggtcctctt gtcgagatc
179
<210> 6
<211> 233
<212> DNA
<213> Artificial sequence
<400> 6
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagccacagt gcatacgtgg gctccaacag gtcctcttgt cgagatctgg taggcgtgta
180
cggtgggagg tctatataag cagagctcgt ttagtgaacc gtcagatcac tag
233
<210> 7
<211> 274
<212> DNA
<213> Artificial sequence
<400> 7
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagccacagt gcatacgtgg gctccaacag gtcctcttgt cgagccacag tgcatacgtg
180
ggctccaaca ggtcctcttg tcgagatctg gtaggcgtgt acggtgggag gtctatataa
240
gcagagctcg tttagtgaac cgtcagatca ctag 274
<210> 8
<211> 292
<212> DNA
<213> Artificial sequence
<400> 8
gtcgacgata tcccacagtg catacgtggg ctccaacagg tcctcttgtc gagccacagt
60
gcatacgtgg gctccaacag gtcctcttgt cgagccacag tgcatacgtg ggctccaaca
120
ggtcctcttg tcgagccaca gtgcatacgt gggctccaac aggtcctctt gtcgagccac
180
agtgcatacg tgggctccaa caggtcctct tgtcgagatc tggtaggcgt gtacggtggg
240
aggtctatat aagcagagct cgtttagtga accgtcagat cactaggcta gc
292
<210> 9
<211> 237
<212> PRT
<213> Artificial sequence
<400> 9

Asp Ile Gln Leu Thr Gln Ser Pro Ser Ser Leu Ser Ala Ser Val Gly
1               5                   10                  15
Asp Arg Val Thr Ile Thr Cys Ser Ala Ser Ser Ser Val Arg Phe Ile
            20                  25                  30
His Trp Tyr Gln Gln Lys Pro Gly Lys Ala Pro Lys Arg Leu Ile Tyr
        35                  40                  45
Asp Thr Ser Lys Leu Ala Ser Gly Val Pro Ser Arg Phe Ser Gly Ser
    50                  55                  60
Gly Ser Gly Thr Asp Phe Thr Leu Thr Ile Ser Ser Leu Gln Pro Glu
65                  70                  75                  80
Asp Phe Ala Thr Tyr Tyr Cys Gln Gln Trp Ser Ser Ser Pro Phe Thr
                85                  90                  95
Phe Gly Gln Gly Thr Lys Val Glu Ile Lys Gly Ser Thr Ser Gly Ser
            100                 105                 110
Gly Lys Pro Gly Ser Gly Glu Gly Ser Thr Lys Gly Ser Glu Val Gln
        115                 120                 125
Leu Val Glu Ser Gly Gly Gly Leu Val Gln Pro Gly Gly Ser Leu Arg
    130                 135                 140
Leu Ser Cys Ala Ala Ser Gly Phe Asn Ile Lys Asp Tyr Tyr Ile His
145                 150                 155                 160
Trp Val Arg Gln Ala Pro Gly Lys Gly Leu Glu Trp Val Ala Trp Ile
                165                 170                 175
Asp Pro Glu Asn Gly Asp Thr Glu Phe Val Pro Lys Phe Gln Gly Arg
            180                 185                 190
Ala Thr Ile Ser Ala Asp Thr Ser Lys Asn Thr Ala Tyr Leu Gln Met
        195                 200                 205
Asn Ser Leu Arg Ala Glu Asp Thr Ala Val Tyr Tyr Cys Lys Thr Gly
    210                 215                 220
Gly Phe Trp Gly Gln Gly Thr Leu Val Thr Val Ser Ser
225                 230                 235
<210> 10
<211> 243
<212> PRT
<213> Artificial sequence
<400> 10
Asp Ile Gln Met Thr Gln Ser Pro Ser Ser Leu Ser Ala Ser Val Gly
1               5                   10                  15
Asp Arg Val Thr Ile Thr Cys Ser Thr Ser Ser Ser Val Ser Tyr Met
            20                  25                  30
His Trp Tyr Gln Gln Lys Pro Gly Lys Ala Pro Arg Leu Leu Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Phe Thr Phe Thr Ile Ser Ser Leu Gln Pro Glu
65                  70                  75                  80
Asp Ile Ala Thr Tyr Tyr Cys His Gln Trp Ser Ser Tyr Pro Thr Phe
                85                  90                  95
Gly Gln Gly Thr Lys Val Glu Ile Lys Gly Ser Thr Ser Gly Ser Gly
            100                 105                 110
Lys Pro Gly Ser Gly Glu Gly Ser Thr Lys Gly Gln Val Gln Leu Gln
        115                 120                 125
Glu Ser Gly Pro Gly Leu Val Arg Pro Ser Gln Thr Leu Ser Leu Thr
    130                 135                 140
Cys Thr Val Ser Gly Phe Thr Ile Ser Ser Gly Tyr Ser Trp His Trp
145                 150                 155                 160
Val Arg Gln Pro Pro Gly Arg Gly Leu Glu Trp Ile Gly Tyr Ile Gln
                165                 170                 175
Tyr Ser Gly Ile Thr Asn Tyr Asn Pro Ser Leu Lys Ser Arg Val Thr

180                 185                 190
Met Leu Val Asp Thr Ser Lys Asn Gln Phe Ser Leu Arg Leu Ser Ser
        195                 200                 205
Val Thr Ala Ala Asp Thr Ala Val Tyr Tyr Cys Ala Arg Glu Asp Tyr
    210                 215                 220
Asp Tyr His Trp Tyr Phe Asp Val Trp Gly Gln Gly Ser Thr Val Thr
225                 230                 235                 240
Val Ser Ser
<210> 11
<211> 243
<212> PRT
<213> Artificial sequence
<400> 11
Gln Ile Gln Leu Val Gln Ser Gly Pro Glu Leu Lys Lys Pro Gly Glu
1               5                   10                  15
Thr Val Lys Ile Ser Cys Lys Ala Ser Gly Tyr Thr Phe Thr Asp Tyr
            20                  25                  30
Ser Ile Asn Trp Val Lys Arg Ala Pro Gly Lys Gly Leu Lys Trp Met
        35                  40                  45
Gly Trp Ile Asn Thr Glu Thr Arg Glu Pro Ala Tyr Ala Tyr Asp Phe
    50                  55                  60
Arg Gly Arg Phe Ala Phe Ser Leu Glu Thr Ser Ala Ser Thr Ala Tyr
65                  70                  75                  80
Leu Gln Ile Asn Asn Leu Lys Tyr Glu Asp Thr Ala Thr Tyr Phe Cys
                85                  90                  95
Ala Leu Asp Tyr Ser Tyr Ala Met Asp Tyr Trp Gly Gln Gly Thr Ser
            100                 105                 110
Val Thr Val Ser Ser Gly Gly Gly Gly Ser Gly Gly Gly Gly Ser Gly
        115                 120                 125
Gly Gly Gly Ser Asp Ile Val Leu Thr Gln Ser Pro Ala Ser Leu Ala
    130                 135                 140
Met Ser Leu Gly Lys Arg Ala Thr Ile Ser Cys Arg Ala Ser Glu Ser
145                 150                 155                 160
Val Ser Val Ile Gly Ala His Leu Ile His Trp Tyr Gln Gln Lys Pro
                165                 170                 175
Gly Gln Pro Pro Lys Leu Leu Ile Tyr Leu Ala Ser Asn Leu Glu Thr
            180                 185                 190
Gly Val Pro Ala Arg Phe Ser Gly Ser Gly Ser Gly Thr Asp Phe Thr
        195                 200                 205
Leu Thr Ile Asp Pro Val Glu Glu Asp Asp Val Ala Ile Tyr Ser Cys
    210                 215                 220
Leu Gln Ser Arg Ile Phe Pro Arg Thr Phe Gly Gly Gly Thr Lys Leu
225                 230                 235                 240
Glu Ile Lys
<210> 12
<211> 245
<212> PRT
<213> Artificial sequence
<400> 12
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln

65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Arg
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser
                245
<210> 13
<211> 240
<212> PRT
<213> Artificial sequence
<400> 13
Gln Val Gln Leu Gln Glu Ser Gly Pro Gly Leu Val Arg Pro Ser Gln
1               5                   10                  15
Thr Leu Ser Leu Thr Cys Thr Val Ser Gly Phe Thr Ile Ser Ser Gly
            20                  25                  30
Tyr Ser Trp His Trp Val Arg Gln Pro Pro Gly Arg Gly Leu Glu Trp
        35                  40                  45
Ile Gly Tyr Ile Gln Tyr Ser Gly Ile Thr Asn Tyr Asn Pro Ser Leu
    50                  55                  60
Lys Ser Arg Val Thr Met Leu Val Asp Thr Ser Lys Asn Gln Phe Ser
65                  70                  75                  80
Leu Arg Leu Ser Ser Val Thr Ala Ala Asp Thr Ala Val Tyr Tyr Cys
                85                  90                  95
Ala Arg Glu Asp Tyr Asp Tyr His Trp Tyr Phe Asp Val Trp Gly Gln
            100                 105                 110
Gly Ser Thr Val Thr Val Ser Ser Gly Gly Gly Gly Ser Gly Gly Gly
        115                 120                 125
Gly Ser Gly Gly Gly Gly Ser Asp Ile Gln Met Thr Gln Ser Pro Ser
    130                 135                 140
Ser Leu Ser Ala Ser Val Gly Asp Arg Val Thr Ile Thr Cys Ser Thr
145                 150                 155                 160
Ser Ser Ser Val Ser Tyr Met His Trp Tyr Gln Gln Lys Pro Gly Lys
                165                 170                 175
Ala Pro Arg Leu Leu Ile Tyr Ser Thr Ser Asn Leu Ala Ser Gly Val
            180                 185                 190
Pro Ser Arg Phe Ser Gly Ser Gly Ser Gly Thr Asp Phe Thr Phe Thr
        195                 200                 205
Ile Ser Ser Leu Gln Pro Glu Asp Ile Ala Thr Tyr Tyr Cys His Gln
    210                 215                 220
Trp Ser Ser Tyr Pro Thr Phe Gly Gln Gly Thr Lys Val Glu Ile Lys
225                 230                 235                 240

<210> 14
<211> 112
<212> PRT
<213> Artificial sequence
<400> 14
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Asn Ile Lys Asp Tyr
            20                  25                  30
Tyr Ile His Trp Val Arg Gln Ala Pro Gly Lys Gly Leu Glu Trp Val
        35                  40                  45
Ala Trp Ile Asp Pro Glu Asn Gly Asp Thr Glu Phe Val Pro Lys Phe
    50                  55                  60
Gln Gly Arg Ala Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Lys Thr Gly Gly Phe Trp Gly Gln Gly Thr Leu Val Thr Val Ser Ser
            100                 105                 110
<210> 15
<211> 106
<212> PRT
<213> Artificial sequence
<400> 15
Asp Ile Gln Leu Thr Gln Ser Pro Ser Ser Leu Ser Ala Ser Val Gly
1               5                   10                  15
Asp Arg Val Thr Ile Thr Cys Ser Ala Ser Ser Ser Val Arg Phe Ile
            20                  25                  30
His Trp Tyr Gln Gln Lys Pro Gly Lys Ala Pro Lys Arg Leu Ile Tyr
        35                  40                  45
Asp Thr Ser Lys Leu Ala Ser Gly Val Pro Ser Arg Phe Ser Gly Ser
    50                  55                  60
Gly Ser Gly Thr Asp Phe Thr Leu Thr Ile Ser Ser Leu Gln Pro Glu
65                  70                  75                  80
Asp Phe Ala Thr Tyr Tyr Cys Gln Gln Trp Ser Ser Ser Pro Phe Thr
                85                  90                  95
Phe Gly Gln Gly Thr Lys Val Glu Ile Lys
            100                 105
<210> 16
<211> 120
<212> PRT
<213> Artificial sequence
<400> 16
Gln Val Gln Leu Gln Glu Ser Gly Pro Gly Leu Val Lys Pro Ser Gln
1               5                   10                  15
Thr Leu Ser Leu Thr Cys Thr Val Ser Gly Val Ser Leu Pro Asp Tyr
            20                  25                  30
Gly Val Ser Trp Ile Arg Gln Pro Pro Gly Lys Ala Leu Glu Trp Leu
        35                  40                  45
Gly Val Ile Trp Gly Ser Glu Thr Thr Tyr Tyr Asn Ser Ser Leu Lys
    50                  55                  60
Thr Arg Leu Thr Ile Ser Lys Asp Asn Ser Lys Asn Gln Val Val Leu
65                  70                  75                  80
Thr Met Thr Asn Met Asp Pro Val Asp Thr Ala Thr Tyr Tyr Cys Ala
                85                  90                  95
Lys His Tyr Tyr Tyr Gly Gly Ser Tyr Ala Met Asp Tyr Trp Gly Gln
            100                 105                 110
Gly Ser Ser Val Thr Val Ser Ser

115                 120
<210> 17
<211> 107
<212> PRT
<213> Artificial sequence
<400> 17
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Lys Tyr
            20                  25                  30
Leu Asn Trp Tyr Gln Gln Lys Pro Gly Lys Ala Pro Arg Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Gly Asn Thr Leu Pro Tyr
                85                  90                  95
Thr Phe Gly Gly Gly Thr Arg Leu Glu Ile Lys
            100                 105
<210> 18
<211> 107
<212> PRT
<213> Artificial sequence
<400> 18
Gln Val Gln Leu Gln Glu Ser Gly Pro Gly Leu Val Arg Pro Ser Gln
1               5                   10                  15
Thr Leu Ser Leu Thr Cys Thr Val Ser Gly Phe Thr Ile Ser Ser Gly
            20                  25                  30
Tyr Ser Trp His Trp Val Arg Gln Pro Pro Gly Arg Gly Leu Glu Trp
        35                  40                  45
Ile Gly Tyr Ile Gln Tyr Ser Gly Ile Thr Asn Tyr Asn Pro Ser Leu
    50                  55                  60
Lys Ser Arg Val Thr Met Leu Val Asp Thr Ser Lys Asn Gln Phe Ser
65                  70                  75                  80
Leu Arg Leu Ser Ser Val Thr Ala Ala Asp Thr Ala Val Tyr Tyr Cys
                85                  90                  95
Ala Arg Glu Asp Tyr Asp Tyr His Trp Tyr Phe Asp Val Trp Gly Gln
            100                 105                 110
Gly Ser Thr Val Thr Val Ser Ser
        115                 120
<210> 19
<211> 105
<212> PRT
<213> Artificial sequence
<400>19
Asp Ile Gln Met Thr Gln Ser Pro Ser Ser Leu Ser Ala Ser Val Gly
1               5                   10                  15
Asp Arg Val Thr Ile Thr Cys Ser Thr Ser Ser Ser Val Ser Tyr Met
            20                  25                  30
His Trp Tyr Gln Gln Lys Pro Gly Lys Ala Pro Arg Leu Leu Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Phe Thr Phe Thr Ile Ser Ser Leu Gln Pro Glu
65                  70                  75                  80
Asp Ile Ala Thr Tyr Tyr Cys His Gln Trp Ser Ser Tyr Pro Thr Phe
                85                  90                  95

Gly Gln Gly Thr Lys Val Glu Ile Lys
            100                 105
<210> 20
<211> 47
<212> PRT
<213> Artificial sequence
<400> 20
Lys Pro Thr Thr Thr Pro Ala Pro Arg Pro Pro Thr Pro Ala Pro Thr
1               5                   10                  15
Ile Ala Ser Gln Pro Leu Ser Leu Arg Pro Glu Ala Cys Arg Pro Ala
            20                  25                  30
Ala Gly Gly Ala Val His Thr Arg Gly Leu Asp Phe Ala Cys Asp
        35                  40                  45
<210> 21
<211> 36
<212> PRT
<213> Artificial sequence
<400> 21
Ala Pro Pro Arg Ala Ser Ala Leu Pro Ala Pro Pro Thr Gly Ser Ala
1               5                   10                  15
Leu Pro Asp Pro Gln Thr Ala Ser Ala Leu Pro Asp Pro Pro Ala Ala
            20                  25                  30
Ser Ala Leu Pro
        35
<210> 22
<211> 45
<212> PRT
<213> Artificial sequence
<400> 22
Lys Pro Thr Thr Thr Pro Ala Pro Arg Pro Pro Thr Pro Ala Pro Thr
1               5                   10                  15
Ile Ala Ser Gln Pro Leu Ser Leu Arg Pro Glu Ala Arg Pro Ala Ala
            20                  25                  30
Gly Gly Ala Val His Thr Arg Gly Leu Asp Phe Ala Asp
        35                  40                  45
<210> 23
<211> 228
<212> PRT
<213> Artificial sequence
<400> 23
Glu Ser Lys Tyr Gly Pro Pro Cys Pro Pro Cys Pro Ala Pro Pro Val
1               5                   10                  15
Ala Gly Pro Ser Val Phe Leu Phe Pro Pro Lys Pro Lys Asp Thr Leu
            20                  25                  30
Met Ile Ser Arg Thr Pro Glu Val Thr Cys Val Val Val Asp Val Ser
        35                  40                  45
Gln Glu Asp Pro Glu Val Gln Phe Asn Trp Tyr Val Asp Gly Val Glu
    50                  55                  60
Val His Asn Ala Lys Thr Lys Pro Arg Glu Glu Gln Phe Gln Ser Thr
65                  70                  75                  80
Tyr Arg Val Val Ser Val Leu Thr Val Leu His Gln Asp Trp Leu Asn
                85                  90                  95
Gly Lys Glu Tyr Lys Cys Lys Val Ser Asn Lys Gly Leu Pro Ser Ser
            100                 105                 110
Ile Glu Lys Thr Ile Ser Lys Ala Lys Gly Gln Pro Arg Glu Pro Gln
        115                 120                 125
Val Tyr Thr Leu Pro Pro Ser Gln Glu Glu Met Thr Lys Asn Gln Val
    130                 135                 140

Ser Leu Thr Cys Leu Val Lys Gly Phe Tyr Pro Ser Asp Ile Ala Val
145                 150                 155                 160
Glu Trp Glu Ser Asn Gly Gln Pro Glu Asn Asn Tyr Lys Thr Thr Pro
                165                 170                 175
Pro Val Leu Asp Ser Asp Gly Ser Phe Phe Leu Tyr Ser Arg Leu Thr
            180                 185                 190
Val Asp Lys Ser Arg Trp Gln Glu Gly Asn Val Phe Ser Cys Ser Val
        195                 200                 205
Met His Glu Ala Leu His Asn His Tyr Thr Gln Lys Ser Leu Ser Leu
    210                 215                 220
Ser Leu Gly Lys
225
<210> 24
<211> 229
<212> PRT
<213> Artificial sequence
<400> 24
Glu Ser Lys Tyr Gly Pro Pro Cys Pro Pro Cys Pro Ala Pro Glu Phe
1               5                   10                  15
Leu Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Asn Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly Lys
225
<210> 25
<211> 282
<212> PRT
<213> Artificial sequence
<400> 25
Arg Trp Pro Glu Ser Pro Lys Ala Gln Ala Ser Ser Val Pro Thr Ala
1               5                   10                  15
Gln Pro Gln Ala Glu Gly Ser Leu Ala Lys Ala Thr Thr Ala Pro Ala
            20                  25                  30
Thr Thr Arg Asn Thr Gly Arg Gly Gly Glu Glu Lys Lys Lys Glu Lys
        35                  40                  45

Glu Lys Glu Glu Gln Glu Glu Arg Glu Thr Lys Thr Pro Glu Cys Pro
    50                  55                  60
Ser His Thr Gln Pro Leu Gly Val Tyr Leu Leu Thr Pro Ala Val Gln
65                  70                  75                  80
Asp Leu Trp Leu Arg Asp Lys Ala Thr Phe Thr Cys Phe Val Val Gly
                85                  90                  95
Ser Asp Leu Lys Asp Ala His Leu Thr Trp Glu Val Ala Gly Lys Val
            100                 105                 110
Pro Thr Gly Gly Val Glu Glu Gly Leu Leu Glu Arg His Ser Asn Gly
        115                 120                 125
Ser Gln Ser Gln His Ser Arg Leu Thr Leu Pro Arg Ser Leu Trp Asn
    130                 135                 140
Ala Gly Thr Ser Val Thr Cys Thr Leu Asn His Pro Ser Leu Pro Pro
145                 150                 155                 160
Gln Arg Leu Met Ala Leu Arg Glu Pro Ala Ala Gln Ala Pro Val Lys
                165                 170                 175
Leu Ser Leu Asn Leu Leu Ala Ser Ser Asp Pro Pro Glu Ala Ala Ser
            180                 185                 190
Trp Leu Leu Cys Glu Val Ser Gly Phe Ser Pro Pro Asn Ile Leu Leu
        195                 200                 205
Met Trp Leu Glu Asp Gln Arg Glu Val Asn Thr Ser Gly Phe Ala Pro
    210                 215                 220
Ala Arg Pro Pro Pro Gln Pro Gly Ser Thr Thr Phe Trp Ala Trp Ser
225                 230                 235                 240
Val Leu Arg Val Pro Ala Pro Pro Ser Pro Gln Pro Ala Thr Tyr Thr
                245                 250                 255
Cys Val Val Ser His Glu Asp Ser Arg Thr Leu Leu Asn Ala Ser Arg
            260                 265                 270
Ser Leu Glu Val Ser Tyr Val Thr Asp His
        275                 280
<210> 26
<211> 27
<212> PRT
<213> Artificial sequence
<400> 26
Phe Trp Val Leu Val Val Val Gly Gly Val Leu Ala Cys Tyr Ser Leu
1               5                   10                  15
Leu Val Thr Val Ala Phe Ile Ile Phe Trp Val
            20                  25
<210> 27
<211> 24
<212> PRT
<213> Artificial sequence
<400> 27
Ile Tyr Ile Trp Ala Pro Leu Ala Gly Thr Cys Gly Val Leu Leu Leu
1               5                   10                  15
Ser Leu Val Ile Thr Leu Tyr Cys
            20
<210> 28
<211> 41
<212> PRT
<213> Artificial sequence
<400> 28
Arg Ser Lys Arg Ser Arg Leu Leu His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Ser

35                  40
<210> 29
<211> 42
<212> PRT
<213> Artificial sequence
<400> 29
Val Lys Arg Gly Arg Lys Lys Leu Leu Tyr Ile Phe Lys Gln Pro Phe
1               5                   10                  15
Met Arg Pro Val Gln Thr Thr Gln Glu Glu Asp Gly Cys Ser Cys Arg
            20                  25                  30
Phe Pro Glu Glu Glu Glu Gly Gly Cys Glu Leu Arg
        35                  40
<210> 30
<211> 35
<212> PRT
<213> Artificial sequence
<400> 30
Thr Lys Lys Lys Tyr Ser Ser Ser Val His Asp Pro Asn Gly Glu Tyr
1               5                   10                  15
Met Phe Met Arg Ala Val Asn Thr Ala Lys Lys Ser Arg Leu Thr Asp
            20                  25                  30
Val Thr Leu
        35
<210> 31
<211> 48
<212> PRT
<213> Artificial sequence
<400> 31
Gln Arg Arg Lys Tyr Arg Ser Asn Lys Gly Glu Ser Pro Val Glu Pro
1               5                   10                  15
Ala Glu Pro Cys Arg Tyr Ser Cys Pro Arg Glu Glu Glu Gly Ser Thr
            20                  25                  30
Ile Pro Ile Gln Glu Asp Tyr Arg Lys Pro Glu Pro Ala Cys Ser Pro
        35                  40                  45
<210> 32
<211> 111
<212> PRT
<213> Artificial sequence
<400> 32
Val Lys Phe Ser Arg Ser Ala Asp Ala Pro Ala Tyr Gln Gln Gly Gln
1               5                   10                  15
Asn Gln Leu Tyr Asn Glu Leu Asn Leu Gly Arg Arg Glu Glu Tyr Asp
            20                  25                  30
Val Leu Asp Lys Arg Arg Gly Arg Asp Pro Glu Met Gly Gly Lys Pro
        35                  40                  45
Arg Arg Lys Asn Pro Gln Glu Gly Leu Tyr Asn Glu Leu Gln Lys Asp
    50                  55                  60
Lys Met Ala Glu Ala Tyr Ser Glu Ile Gly Met Lys Gly Glu Arg Arg
65                  70                  75                  80
Arg Gly Lys Gly His Asp Gly Leu Tyr Gln Gly Leu Ser Thr Ala Thr
                85                  90                  95
Lys Asp Thr Tyr Asp Ala Leu His Met Gln Ala Leu Pro Pro Arg
            100                 105                 110
<210> 33
<211> 644
<212> PRT
<213> Artificial sequence
<400> 33

Asp Ile Gln Leu Thr Gln Ser Pro Ser Ser Leu Ser Ala Ser Val Gly
1               5                   10                  15
Asp Arg Val Thr Ile Thr Cys Ser Ala Ser Ser Ser Val Arg Phe Ile
            20                  25                  30
His Trp Tyr Gln Gln Lys Pro Gly Lys Ala Pro Lys Arg Leu Ile Tyr
        35                  40                  45
Asp Thr Ser Lys Leu Ala Ser Gly Val Pro Ser Arg Phe Ser Gly Ser
    50                  55                  60
Gly Ser Gly Thr Asp Phe Thr Leu Thr Ile Ser Ser Leu Gln Pro Glu
65                  70                  75                  80
Asp Phe Ala Thr Tyr Tyr Cys Gln Gln Trp Ser Ser Ser Pro Phe Thr
                85                  90                  95
Phe Gly Gln Gly Thr Lys Val Glu Ile Lys Gly Ser Thr Ser Gly Ser
            100                 105                 110
Gly Lys Pro Gly Ser Gly Glu Gly Ser Thr Lys Gly Ser Glu Val Gln
        115                 120                 125
Leu Val Glu Ser Gly Gly Gly Leu Val Gln Pro Gly Gly Ser Leu Arg
    130                 135                 140
Leu Ser Cys Ala Ala Ser Gly Phe Asn Ile Lys Asp Tyr Tyr Ile His
145                 150                 155                 160
Trp Val Arg Gln Ala Pro Gly Lys Gly Leu Glu Trp Val Ala Trp Ile
                165                 170                 175
Asp Pro Glu Asn Gly Asp Thr Glu Phe Val Pro Lys Phe Gln Gly Arg
            180                 185                 190
Ala Thr Ile Ser Ala Asp Thr Ser Lys Asn Thr Ala Tyr Leu Gln Met
        195                 200                 205
Asn Ser Leu Arg Ala Glu Asp Thr Ala Val Tyr Tyr Cys Lys Thr Gly
    210                 215                 220
Gly Phe Trp Gly Gln Gly Thr Leu Val Thr Val Ser Ser Glu Ser Lys
225                 230                 235                 240
Tyr Gly Pro Pro Cys Pro Pro Cys Pro Ala Pro Pro Val Ala Gly Pro
                245                 250                 255
Ser Val Phe Leu Phe Pro Pro Lys Pro Lys Asp Thr Leu Met Ile Ser
            260                 265                 270
Arg Thr Pro Glu Val Thr Cys Val Val Val Asp Val Ser Gln Glu Asp
        275                 280                 285
Pro Glu Val Gln Phe Asn Trp Tyr Val Asp Gly Val Glu Val His Asn
    290                 295                 300
Ala Lys Thr Lys Pro Arg Glu Glu Gln Phe Gln Ser Thr Tyr Arg Val
305                 310                 315                 320
Val Ser Val Leu Thr Val Leu His Gln Asp Trp Leu Asn Gly Lys Glu
                325                 330                 335
Tyr Lys Cys Lys Val Ser Asn Lys Gly Leu Pro Ser Ser Ile Glu Lys
            340                 345                 350
Thr Ile Ser Lys Ala Lys Gly Gln Pro Arg Glu Pro Gln Val Tyr Thr
        355                 360                 365
Leu Pro Pro Ser Gln Glu Glu Met Thr Lys Asn Gln Val Ser Leu Thr
    370                 375                 380
Cys Leu Val Lys Gly Phe Tyr Pro Ser Asp Ile Ala Val Glu Trp Glu
385                 390                 395                 400
Ser Asn Gly Gln Pro Glu Asn Asn Tyr Lys Thr Thr Pro Pro Val Leu
                405                 410                 415
Asp Ser Asp Gly Ser Phe Phe Leu Tyr Ser Arg Leu Thr Val Asp Lys
            420                 425                 430
Ser Arg Trp Gln Glu Gly Asn Val Phe Ser Cys Ser Val Met His Glu
        435                 440                 445
Ala Leu His Asn His Tyr Thr Gln Lys Ser Leu Ser Leu Ser Leu Gly
    450                 455                 460

Lys Phe Trp Val Leu Val Val Val Gly Gly Val Leu Ala Cys Tyr Ser
465                 470                 475                 480
Leu Leu Val Thr Val Ala Phe Ile Ile Phe Trp Val Arg Ser Lys Arg
                485                 490                 495
Ser Arg Leu Leu His Ser Asp Tyr Met Asn Met Thr Pro Arg Arg Pro
            500                 505                 510
Gly Pro Thr Arg Lys His Tyr Gln Pro Tyr Ala Pro Pro Arg Asp Phe
        515                 520                 525
Ala Ala Tyr Arg Ser Val Lys Phe Ser Arg Ser Ala Asp Ala Pro Ala
    530                 535                 540
Tyr Gln Gln Gly Gln Asn Gln Leu Tyr Asn Glu Leu Asn Leu Gly Arg
545                 550                 555                 560
Arg Glu Glu Tyr Asp Val Leu Asp Lys Arg Arg Gly Arg Asp Pro Glu
                565                 570                 575
Met Gly Gly Lys Pro Arg Arg Lys Asn Pro Gln Glu Gly Leu Tyr Asn
            580                 585                 590
Glu Leu Gln Lys Asp Lys Met Ala Glu Ala Tyr Ser Glu Ile Gly Met
        595                 600                 605
Lys Gly Glu Arg Arg Arg Gly Lys Gly His Asp Gly Leu Tyr Gln Gly
    610                 615                 620
Leu Ser Thr Ala Thr Lys Asp Thr Tyr Asp Ala Leu His Met Gln Ala
625                 630                 635                 640
Leu Pro Pro Arg
<210> 34
<211> 650
<212> PRT
<213> Artificial sequence
<400> 34
Asp Ile Gln Met Thr Gln Ser Pro Ser Ser Leu Ser Ala Ser Val Gly
1               5                   10                  15
Asp Arg Val Thr Ile Thr Cys Ser Thr Ser Ser Ser Val Ser Tyr Met
            20                  25                  30
His Trp Tyr Gln Gln Lys Pro Gly Lys Ala Pro Arg Leu Leu Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Phe Thr Phe Thr Ile Ser Ser Leu Gln Pro Glu
65                  70                  75                  80
Asp Ile Ala Thr Tyr Tyr Cys His Gln Trp Ser Ser Tyr Pro Thr Phe
                85                  90                  95
Gly Gln Gly Thr Lys Val Glu Ile Lys Gly Ser Thr Ser Gly Ser Gly
            100                 105                 110
Lys Pro Gly Ser Gly Glu Gly Ser Thr Lys Gly Gln Val Gln Leu Gln
        115                 120                 125
Glu Ser Gly Pro Gly Leu Val Arg Pro Ser Gln Thr Leu Ser Leu Thr
    130                 135                 140
Cys Thr Val Ser Gly Phe Thr Ile Ser Ser Gly Tyr Ser Trp His Trp
145                 150                 155                 160
Val Arg Gln Pro Pro Gly Arg Gly Leu Glu Trp Ile Gly Tyr Ile Gln
                165                 170                 175
Tyr Ser Gly Ile Thr Asn Tyr Asn Pro Ser Leu Lys Ser Arg Val Thr
            180                 185                 190
Met Leu Val Asp Thr Ser Lys Asn Gln Phe Ser Leu Arg Leu Ser Ser
        195                 200                 205
Val Thr Ala Ala Asp Thr Ala Val Tyr Tyr Cys Ala Arg Glu Asp Tyr
    210                 215                 220
Asp Tyr His Trp Tyr Phe Asp Val Trp Gly Gln Gly Ser Thr Val Thr
225                 230                 235                 240

Val Ser Ser Glu Ser Lys Tyr Gly Pro Pro Cys Pro Pro Cys Pro Ala
                245                 250                 255
Pro Pro Val Ala Gly Pro Ser Val Phe Leu Phe Pro Pro Lys Pro Lys
            260                 265                 270
Asp Thr Leu Met Ile Ser Arg Thr Pro Glu Val Thr Cys Val Val Val
        275                 280                 285
Asp Val Ser Gln Glu Asp Pro Glu Val Gln Phe Asn Trp Tyr Val Asp
    290                 295                 300
Gly Val Glu Val His Asn Ala Lys Thr Lys Pro Arg Glu Glu Gln Phe
305                 310                 315                 320
Gln Ser Thr Tyr Arg Val Val Ser Val Leu Thr Val Leu His Gln Asp
                325                 330                 335
Trp Leu Asn Gly Lys Glu Tyr Lys Cys Lys Val Ser Asn Lys Gly Leu
            340                 345                 350
Pro Ser Ser Ile Glu Lys Thr Ile Ser Lys Ala Lys Gly Gln Pro Arg
        355                 360                 365
Glu Pro Gln Val Tyr Thr Leu Pro Pro Ser Gln Glu Glu Met Thr Lys
    370                 375                 380
Asn Gln Val Ser Leu Thr Cys Leu Val Lys Gly Phe Tyr Pro Ser Asp
385                 390                 395                 400
Ile Ala Val Glu Trp Glu Ser Asn Gly Gln Pro Glu Asn Asn Tyr Lys
                405                 410                 415
Thr Thr Pro Pro Val Leu Asp Ser Asp Gly Ser Phe Phe Leu Tyr Ser
            420                 425                 430
Arg Leu Thr Val Asp Lys Ser Arg Trp Gln Glu Gly Asn Val Phe Ser
        435                 440                 445
Cys Ser Val Met His Glu Ala Leu His Asn His Tyr Thr Gln Lys Ser
    450                 455                 460
Leu Ser Leu Ser Leu Gly Lys Phe Trp Val Leu Val Val Val Gly Gly
465                 470                 475                 480
Val Leu Ala Cys Tyr Ser Leu Leu Val Thr Val Ala Phe Ile Ile Phe
                485                 490                 495
Trp Val Arg Ser Lys Arg Ser Arg Leu Leu His Ser Asp Tyr Met Asn
            500                 505                 510
Met Thr Pro Arg Arg Pro Gly Pro Thr Arg Lys His Tyr Gln Pro Tyr
        515                 520                 525
Ala Pro Pro Arg Asp Phe Ala Ala Tyr Arg Ser Val Lys Phe Ser Arg
    530                 535                 540
Ser Ala Asp Ala Pro Ala Tyr Gln Gln Gly Gln Asn Gln Leu Tyr Asn
545                 550                 555                 560
Glu Leu Asn Leu Gly Arg Arg Glu Glu Tyr Asp Val Leu Asp Lys Arg
                565                 570                 575
Arg Gly Arg Asp Pro Glu Met Gly Gly Lys Pro Arg Arg Lys Asn Pro
            580                 585                 590
Gln Glu Gly Leu Tyr Asn Glu Leu Gln Lys Asp Lys Met Ala Glu Ala
        595                 600                 605
Tyr Ser Glu Ile Gly Met Lys Gly Glu Arg Arg Arg Gly Lys Gly His
    610                 615                 620
Asp Gly Leu Tyr Gln Gly Leu Ser Thr Ala Thr Lys Asp Thr Tyr Asp
625                 630                 635                 640
Ala Leu His Met Gln Ala Leu Pro Pro Arg
                645                 650
<210> 35
<211> 469
<212> PRT
<213> Artificial sequence
<400> 35
Asp Ile Gln Met Thr Gln Ser Pro Ser Ser Leu Ser Ala Ser Val Gly

1                   5                   10                  15
Asp Arg Val Thr Ile Thr Cys Ser Thr Ser Ser Ser Val Ser Tyr Met
                20                  25                  30
His Trp Tyr Gln Gln Lys Pro Gly Lys Ala Pro Arg Leu Leu Ile Tyr
            35                  40                  45
Ser Thr Ser Asn Leu Ala Ser Gly Val Pro Ser Arg Phe Ser Gly Ser
    50                  55                  60
Gly Ser Gly Thr Asp Phe Thr Phe Thr Ile Ser Ser Leu Gln Pro Glu
65                  70                  75                  80
Asp Ile Ala Thr Tyr Tyr Cys His Gln Trp Ser Ser Tyr Pro Thr Phe
                85                  90                  95
Gly Gln Gly Thr Lys Val Glu Ile Lys Gly Ser Thr Ser Gly Ser Gly
            100                 105                 110
Lys Pro Gly Ser Gly Glu Gly Ser Thr Lys Gly Gln Val Gln Leu Gln
        115                 120                 125
Glu Ser Gly Pro Gly Leu Val Arg Pro Ser Gln Thr Leu Ser Leu Thr
    130                 135                 140
Cys Thr Val Ser Gly Phe Thr Ile Ser Ser Gly Tyr Ser Trp His Trp
145                 150                 155                 160
Val Arg Gln Pro Pro Gly Arg Gly Leu Glu Trp Ile Gly Tyr Ile Gln
                165                 170                 175
Tyr Ser Gly Ile Thr Asn Tyr Asn Pro Ser Leu Lys Ser Arg Val Thr
            180                 185                 190
Met Leu Val Asp Thr Ser Lys Asn Gln Phe Ser Leu Arg Leu Ser Ser
        195                 200                 205
Val Thr Ala Ala Asp Thr Ala Val Tyr Tyr Cys Ala Arg Glu Asp Tyr
    210                 215                 220
Asp Tyr His Trp Tyr Phe Asp Val Trp Gly Gln Gly Ser Thr Val Thr
225                 230                 235                 240
Val Ser Ser Lys Pro Thr Thr Thr Pro Ala Pro Arg Pro Pro Thr Pro
                245                 250                 255
Ala Pro Thr Ile Ala Ser Gln Pro Leu Ser Leu Arg Pro Glu Ala Cys
            260                 265                 270
Arg Pro Ala Ala Gly Gly Ala Val His Thr Arg Gly Leu Asp Phe Ala
        275                 280                 285
Cys Asp Ile Tyr Ile Trp Ala Pro Leu Ala Gly Thr Cys Gly Val Leu
    290                 295                 300
Leu Leu Ser Leu Val Ile Thr Leu Tyr Cys Val Lys Arg Gly Arg Lys
305                 310                 315                 320
Lys Leu Leu Tyr Ile Phe Lys Gln Pro Phe Met Arg Pro Val Gln Thr
                325                 330                 335
Thr Gln Glu Glu Asp Gly Cys Ser Cys Arg Phe Pro Glu Glu Glu Glu
            340                 345                 350
Gly Gly Cys Glu Leu Arg Val Lys Phe Ser Arg Ser Ala Asp Ala Pro
        355                 360                 365
Ala Tyr Gln Gln Gly Gln Asn Gln Leu Tyr Asn Glu Leu Asn Leu Gly
    370                 375                 380
Arg Arg Glu Glu Tyr Asp Val Leu Asp Lys Arg Arg Gly Arg Asp Pro
385                 390                 395                 400
Glu Met Gly Gly Lys Pro Arg Arg Lys Asn Pro Gln Glu Gly Leu Tyr
                405                 410                 415
Asn Glu Leu Gln Lys Asp Lys Met Ala Glu Ala Tyr Ser Glu Ile Gly
            420                 425                 430
Met Lys Gly Glu Arg Arg Arg Gly Lys Gly His Asp Gly Leu Tyr Gln
        435                 440                 445
Gly Leu Ser Thr Ala Thr Lys Asp Thr Tyr Asp Ala Leu His Met Gln
    450                 455                 460
Ala Leu Pro Pro Arg

465
<210> 36
<211> 467
<212> PRT
<213> Artificial sequence
<400> 36
Asp Ile Gln Met Thr Gln Ser Pro Ser Ser Leu Ser Ala Ser Val Gly
1               5                   10                  15
Asp Arg Val Thr Ile Thr Cys Ser Thr Ser Ser Ser Val Ser Tyr Met
            20                  25                  30
His Trp Tyr Gln Gln Lys Pro Gly Lys Ala Pro Arg Leu Leu Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Phe Thr Phe Thr Ile Ser Ser Leu Gln Pro Glu
65                  70                  75                  80
Asp Ile Ala Thr Tyr Tyr Cys His Gln Trp Ser Ser Tyr Pro Thr Phe
                85                  90                  95
Gly Gln Gly Thr Lys Val Glu Ile Lys Gly Ser Thr Ser Gly Ser Gly
            100                 105                 110
Lys Pro Gly Ser Gly Glu Gly Ser Thr Lys Gly Gln Val Gln Leu Gln
        115                 120                 125
Glu Ser Gly Pro Gly Leu Val Arg Pro Ser Gln Thr Leu Ser Leu Thr
    130                 135                 140
Cys Thr Val Ser Gly Phe Thr Ile Ser Ser Gly Tyr Ser Trp His Trp
145                 150                 155                 160
Val Arg Gln Pro Pro Gly Arg Gly Leu Glu Trp Ile Gly Tyr Ile Gln
                165                 170                 175
Tyr Ser Gly Ile Thr Asn Tyr Asn Pro Ser Leu Lys Ser Arg Val Thr
            180                 185                 190
Met Leu Val Asp Thr Ser Lys Asn Gln Phe Ser Leu Arg Leu Ser Ser
        195                 200                 205
Val Thr Ala Ala Asp Thr Ala Val Tyr Tyr Cys Ala Arg Glu Asp Tyr
    210                 215                 220
Asp Tyr His Trp Tyr Phe Asp Val Trp Gly Gln Gly Ser Thr Val Thr
225                 230                 235                 240
Val Ser Ser Lys Pro Thr Thr Thr Pro Ala Pro Arg Pro Pro Thr Pro
                245                 250                 255
Ala Pro Thr Ile Ala Ser Gln Pro Leu Ser Leu Arg Pro Glu Ala Arg
            260                 265                 270
Pro Ala Ala Gly Gly Ala Val His Thr Arg Gly Leu Asp Phe Ala Asp
        275                 280                 285
Ile Tyr Ile Trp Ala Pro Leu Ala Gly Thr Cys Gly Val Leu Leu Leu
    290                 295                 300
Ser Leu Val Ile Thr Leu Tyr Cys Val Lys Arg Gly Arg Lys Lys Leu
305                 310                 315                 320
Leu Tyr Ile Phe Lys Gln Pro Phe Met Arg Pro Val Gln Thr Thr Gln
                325                 330                 335
Glu Glu Asp Gly Cys Ser Cys Arg Phe Pro Glu Glu Glu Glu Gly Gly
            340                 345                 350
Cys Glu Leu Arg Val Lys Phe Ser Arg Ser Ala Asp Ala Pro Ala Tyr
        355                 360                 365
Gln Gln Gly Gln Asn Gln Leu Tyr Asn Glu Leu Asn Leu Gly Arg Arg
    370                 375                 380
Glu Glu Tyr Asp Val Leu Asp Lys Arg Arg Gly Arg Asp Pro Glu Met
385                 390                 395                 400
Gly Gly Lys Pro Arg Arg Lys Asn Pro Gln Glu Gly Leu Tyr Asn Glu
                405                 410                 415

Leu Gln Lys Asp Lys Met Ala Glu Ala Tyr Ser Glu Ile Gly Met Lys
            420                 425                 430
Gly Glu Arg Arg Arg Gly Lys Gly His Asp Gly Leu Tyr Gln Gly Leu
        435                 440                 445
Ser Thr Ala Thr Lys Asp Thr Tyr Asp Ala Leu His Met Gln Ala Leu
    450                 455                 460
Pro Pro Arg
465
<210> 37
<211> 2220
<212> DNA
<213> Artificial sequence
<400> 37
   ccacagtgca tacgtgggct ccaacaggtc ctcttgtcga gccacagtgc atacgtgggc
   60
   tccaacaggt cctcttgtcg agccacagtg catacgtggg ctccaacagg tcctcttgtc
   120
   gagatctggt aggcgtgtac ggtgggaggt ctatataagc agagctcgtt tagtgaaccg
   180
   tcagatcact aggctagcat ggccctgcca gtgaccgccc tgctgctgcc cctggccctg
   240
   ctgctgcacg cagcacggcc cgacatccag gatatccagc tgacacagtc ccctagctcc
   300
   ctgagcgcct ccgtgggcga cagggtgacc atcacatgca gcgcctctag ctccgtgaga
   360
   ttcatccact ggtaccagca gaagcccggc aaggccccta agaggctgat ctatgatacc
   420
   tctaagctgg ccagcggagt gccctcccgc ttctctggca gcggctccgg caccgacttt
   480
   accctgacaa tctctagcct gcagccagag gattttgcca catactattg ccagcagtgg
   540
   tcctctagcc ccttcacctt tggccagggc acaaaggtgg agatcaaggg atctaccagc
   600
   ggatccggca agcctggatc tggagagggc agcacaaagg gctccgaggt gcagctggtg
   660
   gagtccggag gaggactggt gcagccagga ggatctctga ggctgagctg tgcagcctcc
   720
   ggcttcaaca tcaaggacta ctatatccac tgggtgagac aggcacctgg caagggactg
   780
   gagtgggtgg catggatcga ccctgagaat ggcgataccg agttcgtgcc aaagtttcag
   840
   ggcagggcca caatctctgc cgacaccagc aagaacacag cctacctgca gatgaatagc
   900
   ctgcgcgccg aggataccgc cgtgtactat tgtaagacag gaggattttg gggacagggc
   960
   accctggtga cagtgtcctc tctcgaggag agcaagtacg gccctccctg ccccccttgc
   1020
   cctgcccccg agttcctggg cggacccagc gtgttcctgt tcccccccaa gcccaaggac
   1080
   accctgatga tcagccggac ccccgaggtg acctgtgtgg tggtggacgt gtcccaggag
   1140
   gaccccgagg tccagttcaa ctggtacgtg gacggcgtgg aggtgcacaa cgccaagacc
   1200
   aagccccggg aggagcagtt caatagcacc taccgggtgg tgtccgtgct gaccgtgctg
   1260
   caccaggact ggctgaacgg caaggaatac aagtgtaagg tgtccaacaa gggcctgccc
   1320
   agcagcatcg agaaaaccat cagcaaggcc aagggccagc ctcgggagcc ccaggtgtac
   1380
   accctgcccc ctagccaaga ggagatgacc aagaatcagg tgtccctgac ctgcctggtg
   1440
   aagggcttct accccagcga catcgccgtg gagtgggaga gcaacggcca gcccgagaac
   1500
   aactacaaga ccaccccccc tgtgctggac agcgacggca gcttcttcct gtacagcagg
   1560
   ctgaccgtgg acaagagccg gtggcaggag ggcaacgtct ttagctgctc cgtgatgcac
   1620
   gaggccctgc acaaccacta cacccagaag agcctgtccc tgagcctggg caaggaattc
   1680
   ttctgggtgc tggtcgtggt gggtggcgtg ctggcctgct acagcctgct ggtgacagtg
   1740
   gccttcatca tcttttgggt gaggagcaag cggagcagag gcggccacag cgactacatg
   1800
   aacatgaccc cccggaggcc tggccccacc cggaagcact accagcccta cgcccctccc
   1860
   agggacttcg ccgcctaccg gagccgggtg aagttcagcc ggagcgccga cgcccctgcc
   1920
   taccagcagg gccagagcca gctgtacaac gagctgaacc tgggccggag ggaggagtac
   1980
   gacgtgctgg acaagcggag aggccgggac cctgagatgg gcggcaagcc ccggagaaag
   2040
   aaccctcagg agggcctgta taacgaactg cagaaagaca agatggccga ggcctacagc
   2100
   gagatcggca tgaagggcga gcggcggagg ggcaagggcc acgacggcct gtaccagggc
   2160
   ctgagcaccg ccaccaagga tacctacgac gccctgcaca tgcaggccct gccccctcgc
   2220
<210> 38
<211> 2241
<212> DNA
<213> Artificial sequence
<400> 38
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagatctggt aggcgtgtac ggtgggaggt ctatataagc agagctcgtt tagtgaaccg
180
tcagatcact aggctagcat ggccttacca gtgaccgcct tgctcctgcc gctggccttg
240
ctgctccacg ccgccaggcc ggacatccag atgacccaga gcccaagcag cctgagcgcc
300
agcgtgggtg acagagtgac catcacctgt agtaccagtt cgagtgtaag ttacatgcac
360
tggtaccagc agaagccagg taaggctcca aggctgctga tctacagcac atccaacctg
420
gcttctggtg tgccaagcag attcagcggt agcggtagcg gtaccgactt caccttcacc
480
atcagcagcc tccagccaga ggacatcgcc acctactact gccatcagtg gagtagttat
540
cccacgttcg gccaagggac caaggtggaa atcaaaggat ccacttccgg ttcaggaaag
600
cccgggagtg gtgaaggtag cactaaaggc caggtccagc tgcaggagag cggtccaggt
660

cttgtgagac ctagccagac cctgagcctg acctgcaccg tgtctggctt caccatcagc
720
agtggttata gctggcactg ggtgagacag ccacctggac gaggtcttga gtggattgga
780
tacatacagt acagtggtat cactaactac aacccctctc tcaaaagtag agtgacaatg
840
ctggtagaca ccagcaagaa ccagttcagc ctgagactca gcagcgtgac agccgccgac
900
accgcggtct attattgtgc aagagaagac tatgattacc actggtactt cgatgtctgg
960
ggtcaaggca gcacggtcac cgtctcctca ggtgcggccg ccctcgagga gagcaagtac
1020
ggccctccct gccccccttg ccctgccccc gagttcctgg gcggacccag cgtgttcctg
1080
ttccccccca agcccaagga caccctgatg atcagccgga cccccgaggt gacctgtgtg
1140
gtggtggacg tgtcccagga ggaccccgag gtccagttca actggtacgt ggacggcgtg
1200
gaggtgcaca acgccaagac caagccccgg gaggagcagt tcaatagcac ctaccgggtg
1260
gtgtccgtgc tgaccgtgct gcaccaggac tggctgaacg gcaaggaata caagtgtaag
1320
gtgtccaaca agggcctgcc cagcagcatc gagaaaacca tcagcaaggc caagggccag
1380
cctcgggagc cccaggtgta caccctgccc cctagccaag aggagatgac caagaatcag
1440
gtgtccctga cctgcctggt gaagggcttc taccccagcg acatcgccgt ggagtgggag
1500
agcaacggcc agcccgagaa caactacaag accacccccc ctgtgctgga cagcgacggc
1560
agcttcttcc tgtacagcag gctgaccgtg gacaagagcc ggtggcagga gggcaacgtc
1620
tttagctgct ccgtgatgca cgaggccctg cacaaccact acacccagaa gagcctgtcc
1680
ctgagcctgg gcaaggaatt cttctgggtg ctggtcgtgg tgggtggcgt gctggcctgc
1740
tacagcctgc tggtgacagt ggccttcatc atcttttggg tgaggagcaa gcggagcaga
1800
ggcggccaca gcgactacat gaacatgacc ccccggaggc ctggccccac ccggaagcac
1860
taccagccct acgcccctcc cagggacttc gccgcctacc ggagccgggt gaagttcagc
1920
cggagcgccg acgcccctgc ctaccagcag ggccagagcc agctgtacaa cgagctgaac
1980
ctgggccgga gggaggagta cgacgtgctg gacaagcgga gaggccggga ccctgagatg
2040
ggcggcaagc cccggagaaa gaaccctcag gagggcctgt ataacgaact gcagaaagac
2100
aagatggccg aggcctacag cgagatcggc atgaagggcg agcggcggag gggcaagggc
2160
cacgacggcc tgtaccaggg cctgagcacc gccaccaagg atacctacga cgccctgcac
2220
atgcaggccc tgccccctcg c
2241
<210> 39
<211> 1677
<212> DNA
<213> Artificial sequence

<400> 39
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagatctggt aggcgtgtac ggtgggaggt ctatataagc agagctcgtt tagtgaaccg
180
tcagatcact aggctagcat ggccttacca gtgaccgcct tgctcctgcc gctggccttg
240
ctgctccacg ccgccaggcc ggacatccag atgacccaga gcccaagcag cctgagcgcc
300
agcgtgggtg acagagtgac catcacctgt agtaccagtt cgagtgtaag ttacatgcac
360
tggtaccagc agaagccagg taaggctcca aggctgctga tctacagcac atccaacctg
420
gcttctggtg tgccaagcag attcagcggt agcggtagcg gtaccgactt caccttcacc
480
atcagcagcc tccagccaga ggacatcgcc acctactact gccatcagtg gagtagttat
540
cccacgttcg gccaagggac caaggtggaa atcaaaggat ccacttccgg ttcaggaaag
600
cccgggagtg gtgaaggtag cactaaaggc caggtccagc tgcaggagag cggtccaggt
660
cttgtgagac ctagccagac cctgagcctg acctgcaccg tgtctggctt caccatcagc
720
agtggttata gctggcactg ggtgagacag ccacctggac gaggtcttga gtggattgga
780
tacatacagt acagtggtat cactaactac aacccctctc tcaaaagtag agtgacaatg
840
ctggtagaca ccagcaagaa ccagttcagc ctgagactca gcagcgtgac agccgccgac
900
accgcggtct attattgtgc aagagaagac tatgattacc actggtactt cgatgtctgg
960
ggtcaaggca gcacggtcac cgtctcctca ggtgcggccg ccctcgagac cacgacgcca
1020
gcgccgcgac caccaacacc ggcgcccacc atcgcgtcgc agcccctgtc cctgcgccca
1080
gaggcgtgcc ggccagcggc ggggggcgca gtgcacacga gggggctgga cttcgcctgt
1140
gatatctaca tctgggcgcc cttggccggg acttgtgggg tccttctcct gtcactggtt
1200
atcacccttt actgcaaacg gggcagaaag aaactcctgt atatattcaa acaaccattt
1260
atgagaccag tacaaactac tcaagaggaa gatggctgta gctgccgatt tccagaagaa
1320
gaagaaggag gatgtgaact gagagtgaag ttcagcagga gcgcagacgc ccccgcgtac
1380
aagcagggcc agaaccagct ctataacgag ctcaatctag gacgaagaga ggagtacgat
1440
gttttggaca agagacgtgg ccgggaccct gagatggggg gaaagccgag aaggaagaac
1500
cctcaggaag gcctgtacaa tgaactgcag aaagataaga tggcggaggc ctacagtgag
1560
attgggatga aaggcgagcg ccggaggggc aaggggcacg atggccttta ccagggtctc
1620
agtacagcca ccaaggacac ctacgacgcc cttcacatgc aggccctgcc ccctcgc
1677
<210> 40

<211> 1689
<212> DNA
<213> Artificial sequence
<400> 40
gatatcccac agtgcatacg tgggctccaa caggtcctct tgtcgagcca cagtgcatac
60
gtgggctcca acaggtcctc ttgtcgagcc acagtgcata cgtgggctcc aacaggtcct
120
cttgtcgaga tctggtaggc gtgtacggtg ggaggtctat ataagcagag ctcgtttagt
180
gaaccgtcag atcactaggc tagcatggcc ttaccagtga ccgccttgct cctgccgctg
240
gccttgctgc tccacgccgc caggccggac atccagatga cccagagccc aagcagcctg
300
agcgccagcg tgggtgacag agtgaccatc acctgtagta ccagttcgag tgtaagttac
360
atgcactggt accagcagaa gccaggtaag gctccaaggc tgctgatcta cagcacatcc
420
aacctggctt ctggtgtgcc aagcagattc agcggtagcg gtagcggtac cgacttcacc
480
ttcaccatca gcagcctcca gccagaggac atcgccacct actactgcca tcagtggagt
540
agttatccca cgttcggcca agggaccaag gtggaaatca aaggatccac ttccggttca
600
ggaaagcccg ggagtggtga aggtagcact aaaggccagg tccagctgca ggagagcggt
660
ccaggtcttg tgagacctag ccagaccctg agcctgacct gcaccgtgtc tggcttcacc
720
atcagcagtg gttatagctg gcactgggtg agacagccac ctggacgagg tcttgagtgg
780
attggataca tacagtacag tggtatcact aactacaacc cctctctcaa aagtagagtg
840
acaatgctgg tagacaccag caagaaccag ttcagcctga gactcagcag cgtgacagcc
900
gccgacaccg cggtctatta ttgtgcaaga gaagactatg attaccactg gtacttcgat
960
gtctggggtc aaggcagcac ggtcaccgtc tcctcaggtg cggccgccct cgagaagcca
1020
accacaaccc cagcaccaag gccacctaca cctgcaccaa ccatcgcaag ccagccactg
1080
tccctgaggc cagaggcaag acctgcagca ggaggcgccg tgcacacacg gggcctggac
1140
ttcgccgatg aattcatcta catctgggcg cccttggccg ggacttgtgg ggtccttctc
1200
ctgtcactgg ttatcaccct ttactgcaaa cggggcagaa agaaactcct gtatatattc
1260
aaacaaccat ttatgagacc agtacaaact actcaagagg aagatggctg tagctgccga
1320
tttccagaag aagaagaagg aggatgtgaa ctgagagtga agttcagcag gagcgcagac
1380
gcccccgcgt acaagcaggg ccagaaccag ctctataacg agctcaatct aggacgaaga
1440
gaggagtacg atgttttgga caagagacgt ggccgggacc ctgagatggg gggaaagccg
1500
agaaggaaga accctcagga aggcctgtac aatgaactgc agaaagataa gatggcggag
1560
gcctacagtg agattgggat gaaaggcgag cgccggaggg gcaaggggca cgatggcctt
1620

taccagggtc tcagtacagc caccaaggac acctacgacg cccttcacat gcaggccctg
1680
cccccctcgc
1689
<210> 41
<211> 2261
<212> DNA
<213> Artificial sequence
<400> 41
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagccacagt gcatacgtgg gctccaacag gtcctcttgt cgagatctgg taggcgtgta
180
cggtgggagg tctatataag cagagctcgt ttagtgaacc gtcagatcac taggctagca
240
tggccctgcc agtgaccgcc ctgctgctgc ccctggccct gctgctgcac gcagcacggc
300
ccgacatcca ggatatccag ctgacacagt cccctagctc cctgagcgcc tccgtgggcg
360
acagggtgac catcacatgc agcgcctcta gctccgtgag attcatccac tggtaccagc
420
agaagcccgg caaggcccct aagaggctga tctatgatac ctctaagctg gccagcggag
480
tgccctcccg cttctctggc agcggctccg gcaccgactt taccctgaca atctctagcc
540
tgcagccaga ggattttgcc acatactatt gccagcagtg gtcctctagc cccttcacct
600
ttggccaggg cacaaaggtg gagatcaagg gatctaccag cggatccggc aagcctggat
660
ctggagaggg cagcacaaag ggctccgagg tgcagctggt ggagtccgga ggaggactgg
720
tgcagccagg aggatctctg aggctgagct gtgcagcctc cggcttcaac atcaaggact
780
actatatcca ctgggtgaga caggcacctg gcaagggact ggagtgggtg gcatggatcg
840
accctgagaa tggcgatacc gagttcgtgc caaagtttca gggcagggcc acaatctctg
900
ccgacaccag caagaacaca gcctacctgc agatgaatag cctgcgcgcc gaggataccg
960
ccgtgtacta ttgtaagaca ggaggatttt ggggacaggg caccctggtg acagtgtcct
1020
ctctcgagga gagcaagtac ggccctccct gccccccttg ccctgccccc gagttcctgg
1080
gcggacccag cgtgttcctg ttccccccca agcccaagga caccctgatg atcagccgga
1140
cccccgaggt gacctgtgtg gtggtggacg tgtcccagga ggaccccgag gtccagttca
1200
actggtacgt ggacggcgtg gaggtgcaca acgccaagac caagccccgg gaggagcagt
1260
tcaatagcac ctaccgggtg gtgtccgtgc tgaccgtgct gcaccaggac tggctgaacg
1320
gcaaggaata caagtgtaag gtgtccaaca agggcctgcc cagcagcatc gagaaaacca
1380
tcagcaaggc caagggccag cctcgggagc cccaggtgta caccctgccc cctagccaag
1440

aggagatgac caagaatcag gtgtccctga cctgcctggt gaagggcttc taccccagcg
1500
acatcgccgt ggagtgggag agcaacggcc agcccgagaa caactacaag accacccccc
1560
ctgtgctgga cagcgacggc agcttcttcc tgtacagcag gctgaccgtg gacaagagcc
1620
ggtggcagga gggcaacgtc tttagctgct ccgtgatgca cgaggccctg cacaaccact
1680
acacccagaa gagcctgtcc ctgagcctgg gcaaggaatt cttctgggtg ctggtcgtgg
1740
tgggtggcgt gctggcctgc tacagcctgc tggtgacagt ggccttcatc atcttttggg
1800
tgaggagcaa gcggagcaga ggcggccaca gcgactacat gaacatgacc ccccggaggc
1860
ctggccccac ccggaagcac taccagccct acgcccctcc cagggacttc gccgcctacc
1920
ggagccgggt gaagttcagc cggagcgccg acgcccctgc ctaccagcag ggccagagcc
1980
agctgtacaa cgagctgaac ctgggccgga gggaggagta cgacgtgctg gacaagcgga
2040
gaggccggga ccctgagatg ggcggcaagc cccggagaaa gaaccctcag gagggcctgt
2100
ataacgaact gcagaaagac aagatggccg aggcctacag cgagatcggc atgaagggcg
2160
agcggcggag gggcaagggc cacgacggcc tgtaccaggg cctgagcacc gccaccaagg
2220
atacctacga cgccctgcac atgcaggccc tgccccctcg c
2261
<210> 42
<211> 2282
<212> DNA
<213> Artificial sequence
<400> 42
   ccacagtgca tacgtgggct ccaacaggtc ctcttgtcga gccacagtgc atacgtgggc
   60
   tccaacaggt cctcttgtcg agccacagtg catacgtggg ctccaacagg tcctcttgtc
   120
   gagccacagt gcatacgtgg gctccaacag gtcctcttgt cgagatctgg taggcgtgta
   180
   cggtgggagg tctatataag cagagctcgt ttagtgaacc gtcagatcac taggctagca
   240
   tggccttacc agtgaccgcc ttgctcctgc cgctggcctt gctgctccac gccgccaggc
   300
   cggacatcca gatgacccag agcccaagca gcctgagcgc cagcgtgggt gacagagtga
   360
   ccatcacctg tagtaccagt tcgagtgtaa gttacatgca ctggtaccag cagaagccag
   420
   gtaaggctcc aaggctgctg atctacagca catccaacct ggcttctggt gtgccaagca
   480
   gattcagcgg tagcggtagc ggtaccgact tcaccttcac catcagcagc ctccagccag
   540
   aggacatcgc cacctactac tgccatcagt ggagtagtta tcccacgttc ggccaaggga
   600
   ccaaggtgga aatcaaagga tccacttccg gttcaggaaa gcccgggagt ggtgaaggta
   660
   gcactaaagg ccaggtccag ctgcaggaga gcggtccagg tcttgtgaga cctagccaga
   720
   ccctgagcct gacctgcacc gtgtctggct tcaccatcag cagtggttat agctggcact
   780
   gggtgagaca gccacctgga cgaggtcttg agtggattgg atacatacag tacagtggta
   840
   tcactaacta caacccctct ctcaaaagta gagtgacaat gctggtagac accagcaaga
   900
   accagttcag cctgagactc agcagcgtga cagccgccga caccgcggtc tattattgtg
   960
   caagagaaga ctatgattac cactggtact tcgatgtctg gggtcaaggc agcacggtca
   1020
   ccgtctcctc aggtgcggcc gccctcgagg agagcaagta cggccctccc tgcccccctt
   1080
   gccctgcccc cgagttcctg ggcggaccca gcgtgttcct gttccccccc aagcccaagg
   1140
   acaccctgat gatcagccgg acccccgagg tgacctgtgt ggtggtggac gtgtcccagg
   1200
   aggaccccga ggtccagttc aactggtacg tggacggcgt ggaggtgcac aacgccaaga
   1260
   ccaagccccg ggaggagcag ttcaatagca cctaccgggt ggtgtccgtg ctgaccgtgc
   1320
   tgcaccagga ctggctgaac ggcaaggaat acaagtgtaa ggtgtccaac aagggcctgc
   1380
   ccagcagcat cgagaaaacc atcagcaagg ccaagggcca gcctcgggag ccccaggtgt
   1440
   acaccctgcc ccctagccaa gaggagatga ccaagaatca ggtgtccctg acctgcctgg
   1500
   tgaagggctt ctaccccagc gacatcgccg tggagtggga gagcaacggc cagcccgaga
   1560
   acaactacaa gaccaccccc cctgtgctgg acagcgacgg cagcttcttc ctgtacagca
   1620
   ggctgaccgt ggacaagagc cggtggcagg agggcaacgt ctttagctgc tccgtgatgc
   1680
   acgaggccct gcacaaccac tacacccaga agagcctgtc cctgagcctg ggcaaggaat
   1740
   tcttctgggt gctggtcgtg gtgggtggcg tgctggcctg ctacagcctg ctggtgacag
   1800
   tggccttcat catcttttgg gtgaggagca agcggagcag aggcggccac agcgactaca
   1860
   tgaacatgac cccccggagg cctggcccca cccggaagca ctaccagccc tacgcccctc
   1920
   ccagggactt cgccgcctac cggagccggg tgaagttcag ccggagcgcc gacgcccctg
   1980
   cctaccagca gggccagagc cagctgtaca acgagctgaa cctgggccgg agggaggagt
   2040
   acgacgtgct ggacaagcgg agaggccggg accctgagat gggcggcaag ccccggagaa
   2100
   agaaccctca ggagggcctg tataacgaac tgcagaaaga caagatggcc gaggcctaca
   2160
   gcgagatcgg catgaagggc gagcggcgga ggggcaaggg ccacgacggc ctgtaccagg
   2220
   gcctgagcac cgccaccaag gatacctacg acgccctgca catgcaggcc ctgccccctc
   2280
   gc 2282
<210> 43
<211> 1724
<212> DNA
<213> Artificial sequence
<400> 43

gatatcccac agtgcatacg tgggctccaa caggtcctct tgtcgagcca cagtgcatac
60
gtgggctcca acaggtcctc ttgtcgagcc acagtgcata cgtgggctcc aacaggtcct
120
cttgtcgagc cacagtgcat acgtgggctc caacaggtcc tcttgtcgag atctggtagg
180
cgtgtacggt gggaggtcta tataagcaga gctcgtttag tgaaccgtca gatcactagg
240
ctagcatggc cttaccagtg accgccttgc tcctgccgct ggccttgctg ctccacgccg
300
ccaggccgga catccagatg acccagagcc caagcagcct gagcgccagc gtgggtgaca
360
gagtgaccat cacctgtagt accagttcga gtgtaagtta catgcactgg taccagcaga
420
agccaggtaa ggctccaagg ctgctgatct acagcacatc caacctggct tctggtgtgc
480
caagcagatt cagcggtagc ggtagcggta ccgacttcac cttcaccatc agcagcctcc
540
agccagagga catcgccacc tactactgcc atcagtggag tagttatccc acgttcggcc
600
aagggaccaa ggtggaaatc aaaggatcca cttccggttc aggaaagccc gggagtggtg
660
aaggtagcac taaaggccag gtccagctgc aggagagcgg tccaggtctt gtgagaccta
720
gccagaccct gagcctgacc tgcaccgtgt ctggcttcac catcagcagt ggttatagct
780
ggcactgggt gagacagcca cctggacgag gtcttgagtg gattggatac atacagtaca
840
gtggtatcac taactacaac ccctctctca aaagtagagt gacaatgctg gtagacacca
900
gcaagaacca gttcagcctg agactcagca gcgtgacagc cgccgacacc gcggtctatt
960
attgtgcaag agaagactat gattaccact ggtacttcga tgtctggggt caaggcagca
1020
cggtcaccgt ctcctcaggt gcggccgccc tcgagaccac gacgccagcg ccgcgaccac
1080
caacaccggc gcccaccatc gcgtcgcagc ccctgtccct gcgcccagag gcgtgccggc
1140
cagcggcggg gggcgcagtg cacacgaggg ggctggactt cgcctgtgat atctacatct
1200
gggcgccctt ggccgggact tgtggggtcc ttctcctgtc actggttatc accctttact
1260
gcaaacgggg cagaaagaaa ctcctgtata tattcaaaca accatttatg agaccagtac
1320
aaactactca agaggaagat ggctgtagct gccgatttcc agaagaagaa gaaggaggat
1380
gtgaactgag agtgaagttc agcaggagcg cagacgcccc cgcgtacaag cagggccaga
1440
accagctcta taacgagctc aatctaggac gaagagagga gtacgatgtt ttggacaaga
1500
gacgtggccg ggaccctgag atggggggaa agccgagaag gaagaaccct caggaaggcc
1560
tgtacaatga actgcagaaa gataagatgg cggaggccta cagtgagatt gggatgaaag
1620
gcgagcgccg gaggggcaag gggcacgatg gcctttacca gggtctcagt acagccacca
1680
aggacaccta cgacgccctt cacatgcagg ccctgccccc tcgc
1724

<210> 44
<211> 1724
<212> DNA
<213> Artificial sequence
<400> 44
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagccacagt gcatacgtgg gctccaacag gtcctcttgt cgagatctgg taggcgtgta
180
cggtgggagg tctatataag cagagctcgt ttagtgaacc gtcagatcac taggctagca
240
tggccttacc agtgaccgcc ttgctcctgc cgctggcctt gctgctccac gccgccaggc
300
cggacatcca gatgacccag agcccaagca gcctgagcgc cagcgtgggt gacagagtga
360
ccatcacctg tagtaccagt tcgagtgtaa gttacatgca ctggtaccag cagaagccag
420
gtaaggctcc aaggctgctg atctacagca catccaacct ggcttctggt gtgccaagca
480
gattcagcgg tagcggtagc ggtaccgact tcaccttcac catcagcagc ctccagccag
540
aggacatcgc cacctactac tgccatcagt ggagtagtta tcccacgttc ggccaaggga
600
ccaaggtgga aatcaaagga tccacttccg gttcaggaaa gcccgggagt ggtgaaggta
660
gcactaaagg ccaggtccag ctgcaggaga gcggtccagg tcttgtgaga cctagccaga
720
ccctgagcct gacctgcacc gtgtctggct tcaccatcag cagtggttat agctggcact
780
gggtgagaca gccacctgga cgaggtcttg agtggattgg atacatacag tacagtggta
840
tcactaacta caacccctct ctcaaaagta gagtgacaat gctggtagac accagcaaga
900
accagttcag cctgagactc agcagcgtga cagccgccga caccgcggtc tattattgtg
960
caagagaaga ctatgattac cactggtact tcgatgtctg gggtcaaggc agcacggtca
1020
ccgtctcctc aggtgcggcc gccctcgaga agccaaccac aaccccagca ccaaggccac
1080
ctacacctgc accaaccatc gcaagccagc cactgtccct gaggccagag gcaagacctg
1140
cagcaggagg cgccgtgcac acacggggcc tggacttcgc cgatgaattc atctacatct
1200
gggcgccctt ggccgggact tgtggggtcc ttctcctgtc actggttatc accctttact
1260
gcaaacgggg cagaaagaaa ctcctgtata tattcaaaca accatttatg agaccagtac
1320
aaactactca agaggaagat ggctgtagct gccgatttcc agaagaagaa gaaggaggat
1380
gtgaactgag agtgaagttc agcaggagcg cagacgcccc cgcgtacaag cagggccaga
1440
accagctcta taacgagctc aatctaggac gaagagagga gtacgatgtt ttggacaaga
1500
gacgtggccg ggaccctgag atggggggaa agccgagaag gaagaaccct caggaaggcc
1560

tgtacaatga actgcagaaa gataagatgg cggaggccta cagtgagatt gggatgaaag
1620
gcgagcgccg gaggggcaag gggcacgatg gcctttacca gggtctcagt acagccacca
1680
aggacaccta cgacgccctt cacatgcagg ccctgccccc tcgc
1724
<210> 45
<211> 1599
<212> DNA
<213> Artificial sequence
<400> 45
atggccctgc cagtgaccgc cctgctgctg cccctggccc tgctgctgca cgcagcacgg
60
cccgacatcc aggatattca gctgacccag agcccgagca gcctgagcgc gagcgtgggc
120
gatcgcgtga ccattacctg cagcgcgagc agcagcgtgc gctttattca ttggtatcag
180
cagaaaccgg gcaaagcgcc gaaacgcctg atttatgata ccagcaaact ggcgagcggc
240
gtgccgagcc gctttagcgg cagcggcagc ggcaccgatt ttaccctgac cattagcagc
300
ctgcagccgg aagattttgc gacctattat tgccagcagt ggagcagcag cccgtttacc
360
tttggccagg gcaccaaagt ggaaattaaa ggcagcacca gcggcagcgg caaaccgggc      420
agcggcgaag gcagcaccaa aggcagcgaa gtgcagctgg tggaaagcgg cggcggcctg      480
gtgcagccgg gcggcagcct gcgcctgagc tgcgcggcga gcggctttaa cattaaagat
540
tattatattc attgggtgcg ccaggcgccg ggcaaaggcc tggaatgggt ggcgtggatt
600
gatccggaaa acggcgatac cgaatttgtg ccgaaatttc agggccgcgc gaccattagc
660
gcggatacca gcaaaaacac cgcgtatctg cagatgaaca gcctgcgcgc ggaagatacc
720
gcggtgtatt attgcaaaac cggcggcttt tggggccagg gcaccctggt gaccgtgagc
780
tctctcgaga ccaccacccc ggcgccgcgc ccgccgaccc cggcgccgac cattgcgagc
840
cagccgctga gcctgcgccc ggaagcgtgc cgcccggcgg cgggcggcgc ggtgcatacc      900
cgcggcctgg attttgcgtg cgatgaattc ttttgggtgc tggtggtggt gggcggcgtg
960
ctggcgtgct atagcctgct ggtgaccgtg gcgtttatta ttttttgggt gcgcagcaaa
1020
cgcagccgcg gcggccatag cgattatatg aacatgaccc cgcgccgccc gggcccgacc
1080
cgcaaacatt atcagccgta tgcgccgccg cgcgattttg cggcgtatcg cagcgtgaaa
1140
cgcggccgca aaaaactgct gtatattttt aaacagccgt ttatgcgccc ggtgcagacc
1200
acccaggaag aagatggctg cagctgccgc tttccggaag aagaagaagg cggctgcgaa
1260
ctgcgcgtga aatttagccg cagcgcggat gcgccggcgt atcagcaggg ccagaaccag
1320
ctgtataacg aactgaacct gggccgccgc gaagaatatg atgtgctgga taaacgccgc
1380
ggccgcgatc cggaaatggg cggcaaaccg cgccgcaaaa acccgcagga aggcctgtat
1440
aacgaactgc agaaagataa aatggcggaa gcgtatagcg aaattggcat gaaaggcgaa
1500

cgccgccgcg gcaaaggcca tgatggcctg tatcagggcc tgagcaccgc gaccaaagat
1560
acctatgatg cgctgcatat gcaggcgctg ccgccgcgc                    1599
<210> 46
<211> 1473
<212> DNA
<213> Artificial sequence
<400> 46
atggcgctgc cggtgaccgc gctgctgctg ccgctggcgc tgctgctgca tgcggcgcgc
60
ccggatattc agatgaccca gagcccgagc agcctgagcg cgagcgtggg cgatcgcgtg
120
accattacct gccgcgcgag ccaggatatt agcaaatatc tgaactggta tcagcagaaa
180
ccgggcaaag cgccgcgcct gctgatttat cataccagcc gcctgcatag cggcgtgccg
240
agccgcttta gcggcagcgg cagcggcacc gattataccc tgaccattag cagcctgcag
300
ccggaagatt ttgcgaccta ttattgccag cagggcaaca ccctgccgta tacctttggc
360
ggcggcaccc gcctggaaat taaaggcagc accagcggca gcggcaaacc gggcagcggc    420
gaaggcagca ccaaaggcca ggtgcagctg caggaaagcg gcccgggcct ggtgaaaccg    480
agccagaccc tgagcctgac ctgcaccgtg agcggcgtga gcctgccgga ttatggcgtg
540
agctggattc gccagccgcc gggcaaagcg ctggaatggc tgggcgtgat ttggggcagc
600
gaaaccacct attataacag cagcctgaaa acccgcctga ccattagcaa agataacagc
660
aaaaaccagg tggtgctgac catgaccaac atggatccgg tggataccgc gacctattat
720
tgcgcgaaac attattatta tggcggcagc tatgcgatgg attattgggg ccagggcagc
780
agcgtgaccg tgagcagcct ggaaaccacc accccggcgc cgcgcccgcc gaccccggcg    840
ccgaccattg cgagccagcc gctgagcctg cgcccggaag cgtgccgccc ggcggcgggc    900
ggcgcggtgc atacccgcgg cctggatttt gcgtgcgata tttatatttg ggcgccgctg
960
gcgggcacct gcggcgtgct gctgctgagc ctggtgatta ccctgtattg caaacgcggc
1020
cgcaaaaaac tgctgtatat ttttaaacag ccgtttatgc gcccggtgca gaccacccag
1080
gaagaagatg gctgcagctg ccgctttccg gaagaagaag aaggcggctg cgaactgcgc
1140
gtgaaattta gccgcagcgc ggatgcgccg gcgtataaac agggccagaa ccagctgtat
1200
aacgaactga acctgggccg ccgcgaagaa tatgatgtgc tggataaacg ccgcggccgc
1260
gatccggaaa tgggcggcaa accgcgccgc aaaaacccgc aggaaggcct gtataacgaa
1320
ctgcagaaag ataaaatggc ggaagcgtat agcgaaattg gcatgaaagg cgaacgccgc
1380
cgcggcaaag gccatgatgg cctgtatcag ggcctgagca ccgcgaccaa agatacctat
1440
gatgcgctgc atatgcaggc gctgccgccg cgc                              1473
<210> 47
<211> 1980
<212> DNA
<213> Artificial sequence
<400> 47

gatattcaga tgacccagag cccgagcagc ctgagcgcga gcgtgggcga tcgcgtgacc
60
attacctgca gcaccagcag cagcgtgagc tatatgcatt ggtatcagca gaaaccgggc
120
aaagcgccgc gcctgctgat ttatagcacc agcaacctgg cgagcggcgt gccgagccgc
180
tttagcggca gcggcagcgg caccgatttt acctttacca ttagcagcct gcagccggaa
240
gatattgcga cctattattg ccatcagtgg agcagctatc cgacctttgg ccagggcacc
300
aaagtggaaa ttaaaggcag caccagcggc agcggcaaac cgggcagcgg cgaaggcagc
360
accaaaggcc aggtgcagct gcaggaaagc ggcccgggcc tggtgcgccc gagccagacc
420
ctgagcctga cctgcaccgt gagcggcttt accattagca gcggctatag ctggcattgg
480
gtgcgccagc cgccgggccg cggcctggaa tggattggct atattcagta tagcggcatt
540
accaactata acccgagcct gaaaagccgc gtgaccatgc tggtggatac cagcaaaaac
600
cagtttagcc tgcgcctgag cagcgtgacc gcggcggata ccgcggtgta ttattgcgcg
660
cgcgaagatt atgattatca ttggtatttt gatgtgtggg gccagggcag caccgtgacc
720
gtgagcagcg gcgcggcggc gctggaagaa agcaaatatg gcccgccgtg cccgccgtgc
780
ccggcgccgg aatttctggg cggcccgagc gtgtttctgt ttccgccgaa accgaaagat
840
accctgatga ttagccgcac cccggaagtg acctgcgtgg tggtggatgt gagccaggaa
900
gatccggaag tgcagtttaa ctggtatgtg gatggcgtgg aagtgcataa cgcgaaaacc
960
aaaccgcgcg aagaacagtt taacagcacc tatcgcgtgg tgagcgtgct gaccgtgctg
1020
catcaggatt ggctgaacgg caaagaatat aaatgcaaag tgagcaacaa aggcctgccg
1080
agcagcattg aaaaaaccat tagcaaagcg aaaggccagc cgcgcgaacc gcaggtgtat
1140
accctgccgc cgagccagga agaaatgacc aaaaaccagg tgagcctgac ctgcctggtg
1200
aaaggctttt atccgagcga tattgcggtg gaatgggaaa gcaacggcca gccggaaaac
1260
aactataaaa ccaccccgcc ggtgctggat agcgatggca gcttttttct gtatagccgc
1320
ctgaccgtgg ataaaagccg ctggcaggaa ggcaacgtgt ttagctgcag cgtgatgcat
1380
gaagcgctgc ataaccatta tacccagaaa agcctgagcc tgagcctggg caaagaattt
1440
ttttgggtgc tggtggtggt gggcggcgtg ctggcgtgct atagcctgct ggtgaccgtg
1500
gcgtttatta ttttttgggt gcgcagcaaa cgcagccgcg gcggccatag cgattatatg
1560
aacatgaccc cgcgccgccc gggcccgacc cgcaaacatt atcagccgta tgcgccgccg
1620
cgcgattttg cggcgtatcg cagccgcgtg aaatttagcc gcagcgcgga tgcgccggcg
1680
tatcagcagg gccagagcca gctgtataac gaactgaacc tgggccgccg cgaagaatat
1740

gatgtgctgg ataaacgccg cggccgcgat ccggaaatgg gcggcaaacc gcgccgcaaa
1800
aacccgcagg aaggcctgta taacgaactg cagaaagata aaatggcgga agcgtatagc
1860
gaaattggca tgaaaggcga acgccgccgc ggcaaaggcc atgatggcct gtatcagggc
1920
ctgagcaccg cgaccaaaga tacctatgat gcgctgcata tgcaggcgct gccgccgcgc
1980
<210> 48
<211> 399
<212> DNA
<213> Artificial sequence
<400> 48
tttatttagt ctccagaaaa aggggggaat gaaagacccc acctgtaggt ttggcaagct
60
aggatcaagg ttaggaacag agagacagca gaatatgggc caaacaggat atctgtggta
120
agcagttcct gccccggctc agggccaaga acagttggaa cagcagaata tgggccaaac
180
aggatatctg tggtaagcag ttcctgcccc ggctcagggc caagaacaga tggtccccag
240
atgcggtccc gccctcagca gtttctagag aaccatcaga tgtttccagg gtgccccaag
300
gacctgaaat gaccctgtgc cttatttgaa ctaaccaatc agttcgcttc tcgcttctgt
360
tcgcgcgctt ctgctccccg agctcaataa aagagccca
399
<210> 49
<211> 118
<212> DNA
<213> Artificial sequence
<400> 49
acccctctct ccagccacta agccagttgc tccctcggct gacggctgca cgcgaggcct
60
ccgaacgtct tacgccttgt ggcgcgcccg tccttgtccc gggtgtgatg gcggggtg
118
<210> 50
<211> 221
<212> DNA
<213> Artificial sequence
<400> 50
acccctctct ccagccacta agccagttgc tccctcggct gacggctgca cgcgaggcct
60
ccgaacgtct tacgccttgt ggcgcgcccg tccttgtccc gggtgtgatg gcggggtgtg
120
gggcggaggg cgtggcgggg aagggccggc gacgagagcc gcgcgggacg actcgtcggc
180
gataaccggt gtcgggtagc gccagccgcg cgacggtaac g
221
<210> 51
<211> 324
<212> DNA
<213> Artificial sequence
<400> 51
acccctctct ccagccacta agccagttgc tccctcggct gacggctgca cgcgaggcct
60
ccgaacgtct tacgccttgt ggcgcgcccg tccttgtccc gggtgtgatg gcggggtgtg
120

gggcggaggg cgtggcgggg aagggccggc gacgagagcc gcgcgggacg actcgtcggc
180
gataaccggt gtcgggtagc gccagccgcg cgacggtaac gagggaccgc gacaggcaga
240
cgctcccatg atcactctgc acgccgaagg caaatagtgc aggccgtgcg gcgcttggcg
300
ttccttggaa gggctgaatc cccg
324
<210> 52
<211> 422
<212> DNA
<213> Artificial sequence
<400> 52
acccctctct ccagccacta agccagttgc tccctcggct gacggctgca cgcgaggcct
60
ccgaacgtct tacgccttgt ggcgcgcccg tccttgtccc gggtgtgatg gcggggtgtg
120
gggcggaggg cgtggcgggg aagggccggc gacgagagcc gcgcgggacg actcgtcggc
180
gataaccggt gtcgggtagc gccagccgcg cgacggtaac gagggaccgc gacaggcaga
240
cgctcccatg atcactctgc acgccgaagg caaatagtgc aggccgtgcg gcgcttggcg
300
ttccttggaa gggctgaatc cccgcctcgt ccttcgcagc ggccccccgg gtgttcccat
360
cgccgcttct aggcccactg cgacgcttgc ctgcacttct tacacgctct gggtcccagc
420
cg
422
<210> 53
<211> 1184
<212> DNA
<213> Artificial sequence
<400> 53
cgtgaggctc cggtgcccgt cagtgggcag agcgcacatc gcccacagtc cccgagaagt
60
tggggggagg ggtcggcaat tgaaccggtg cctagagaag gtggcgcggg gtaaactggg
120
aaagtgatgt cgtgtactgg ctccgccttt ttcccgaggg tgggggagaa ccgtatataa
180
gtgcagtagt cgccgtgaac gttctttttc gcaacgggtt tgccgccaga acacaggtaa
240
gtgccgtgtg tggttcccgc gggcctggcc tctttacggg ttatggccct tgcgtgcctt
300
gaattacttc cacctggctg cagtacgtga ttcttgatcc cgagcttcgg gttggaagtg
360
ggtgggagag ttcgaggcct tgcgcttaag gagccccttc gcctcgtgct tgagttgagg
420
cctggcctgg gcgctggggc cgccgcgtgc gaatctggtg gcaccttcgc gcctgtctcg
480
ctgctttcga taagtctcta gccatttaaa atttttgatg acctgctgcg acgctttttt
540
tctggcaaga tagtcttgta aatgcgggcc aagatctgca cactggtatt tcggtttttg
600
gggccgcggg cggcgacggg gcccgtgcgt cccagcgcac atgttcggcg aggcggggcc
660
tgcgagcgcg gccaccgaga atcggacggg ggtagtctca agctggccgg cctgctctgg
720

tgcctggcct cgcgccgccg tgtatcgccc cgccctgggc ggcaaggctg gcccggtcgg
780
caccagttgc gtgagcggaa agatggccgc ttcccggccc tgctgcaggg agctcaaaat
840
ggaggacgcg gcgctcggga gagcgggcgg gtgagtcacc cacacaaagg aaaagggcct
900
ttccgtcctc agccgtcgct tcatgtgact ccacggagta ccgggcgccg tccaggcacc
960
tcgattagtt ctcgagcttt tggagtacgt cgtctttagg ttggggggag gggttttatg
1020
cgatggagtt tccccacact gagtgggtgg agactgaagt taggccagct tggcacttga
1080
tgtaattctc cttggaattt gccctttttg agtttggatc ttggttcatt ctcaagcctc
1140
agacagtggt tcaaagtttt tttcttccat ttcaggtgtc gtga
1184
<210> 54
<211> 399
<212> DNA
<213> Artificial sequence
<400> 54
tttatttagt ctccagaaaa aggggggaat gaaagacccc acctgtaggt ttggcaagct
60
aggatcaagg ttaggaacag agagacagca gaatatgggc caaacaggat atctgtggta
120
agcagttcct gccccggctc agggccaaga acagttggaa cagcagaata tgggccaaac
180
aggatatctg tggtaagcag ttcctgcccc ggctcagggc caagaacaga tggtccccag
240
atgcggtccc gccctcagca gtttctagag aaccatcaga tgtttccagg gtgccccaag
300
gacctgaaat gaccctgtgc cttatttgaa ctaaccaatc agttcgcttc tcgcttctgt
360
tcgcgcgctt ctgctccccg agctcaataa aagagccca
399
<210> 55
<211> 150
<212> PRT
<213> Artificial sequence
<400> 55
<210> 56
<211> 717
<212> DNA
<213> Artificial sequence
<400> 56
atgcagatcc cacaggcgcc ctggccagtc gtctgggcgg tgctacaact gggctggcgg
60
ccaggatggt tcttagactc cccagacagg ccctggaacc cccccacctt ctccccagcc
120
ctgctcgtgg tgaccgaagg ggacaacgcc accttcacct gcagcttctc caacacatcg
180
gagagcttcg tgctaaactg gtaccgcatg agccccagca accagacgga caagctggcc
240
gccttccccg aggaccgcag ccagcccggc caggactgcc gcttccgtgt cacacaactg
300
cccaacgggc gtgacttcca catgagcgtg gtcagggccc ggcgcaatga cagcggcacc
360
tacctctgtg gggccatctc cctggccccc aaggcgcaga tcaaagagag cctgcgggca
420
gagctcaggg tgacagagag aagggcagaa gtgcccacag cccaccccag cccctcaccc
480
aggccagccg gccagttcca aaccctggtg ttttgggtgc tggtggtggt tggtggagtc
540
ctggcttgct atagcttgct agtaacagtg gcctttatta ttttctgggt gaggagtaag
600
aggagcaggc tcctgcacag tgactacatg aacatgactc cccgccgccc cgggcccacc
660
cgcaagcatt accagcccta tgccccacca cgcgacttcg cagcctatcg ctcctaa
717
<210> 57
<211> 833
<212> DNA
<213> Artificial sequence
<400> 57
atgcagatcc cacaggcgcc ctggccagtc gtctgggcgg tgctacaact gggctggcgg
60
ccaggatggt tcttagactc cccagacagg ccctggaacc cccccacctt ctccccagcc
120
ctgctcgtgg tgaccgaagg ggacaacgcc accttcacct gcagcttctc caacacatcg
180
gagagcttcg tgctaaactg gtaccgcatg agccccagca accagacgga caagctggcc
240
gccttccccg aggaccgcag ccagcccggc caggactgcc gcttccgtgt cacacaactg
300
cccaacgggc gtgacttcca catgagcgtg gtcagggccc ggcgcaatga cagcggcacc
360
tacctctgtg gggccatctc cctggccccc aaggcgcaga tcaaagagag cctgcgggca
420
gagctcaggg tgacagagag aagggcagaa gtgcccacag cccactgtcc aagtccccta
480
tttcccggac cttctaagcc cttttgggtg ctggtggtgg ttggtggagt cctggcttgc
540
tatagcttgc tagtaacagt ggcctttatt attttctggg tgaggagtaa gaggagcagg
600
ctcctgcaca gtgactacat gaacatgact ccccgccgcc ccgggcccac ccgcaagcat
660

taccagccct atgccccacc acgcgacttc gcagcctatc gctccgttaa acggggcaga
720
aagaaactcc tgtatatatt caaacaacca tttatgagac cagtacaaac tactcaagag
780
gaagatggct tagctgccga tttccagaag aagaagaagg aggatgtgaa ctg
833
<210> 58
<211> 272
<212> DNA
<213> Artificial sequence
<400>58
ccacagtgca tacgtgggct ccaacaggtc ctcttgtcga gccacagtgc atacgtgggc
60
tccaacaggt cctcttgtcg agccacagtg catacgtggg ctccaacagg tcctcttgtc
120
gagccacagt gcatacgtgg gctccaacag gtcctcttgt cgagccacag tgcatacgtg
180
ggctccaaca ggtcctcttg tcgagatctg gtaggcgtgt acggtgggag gtctatataa
240
gcagagctcg magtgaaccg tcagatcact ag                                272

## Description

### Background of the Present Disclosure

### Field of Disclosure

The present disclosure belongs to the technical field of genetic engineering, and particularly relates to a promoter, a CAR construct, a separated nucleic acid molecule, an expression vector, a CAR-T cell and use thereof in preparation of tumor drugs, and a method for improving a killing capacity of a CAR-T cell under an anoxic environment.

### Description of Related Arts

CAR-T is short for chimeric antigen receptor T-cell immunotherapy, which has better performance in hematologic tumors than in solid tumors. The reasons are as follows: first, it is difficult for CAR-T cells to enter solid tumors; second, CAR-T cells cannot function properly in tumor microenvironments even if they are able to enter solid tumors. The above affects the efficacy of CAR-T cells in solid tumor therapy. In addition, solid tumors have a high heterogeneous degree, and usually, targets for solid tumors are also expressed in normal tissues. Therefore, there are safety problems such as off-targeting. Although available constructs such as fourth-generation CARs, dual CARs (CAR-T with dual antigens), and iCARs having activation inhibition functions are expected to improve the efficacy and safety of solid tumor therapy, these CAR constructs still have problems such as safety or difficulty in activation. Therefore, it is necessary to construct a CAR construct that specifically initiates activation in a tumor microenvironment.

Acidity and hypoxia are two major physical factors in a tumor microenvironment. Most cells in solid tumors are under a hypoxic environment. Hypoxia alters sugar metabolic pathways of tumor cells, produces a large amount of lactic acid, and causes acidic features in the tumor microenvironment. The acidity can reduce apoptosis of the tumor cells, enhance their proliferation and growth, and help their metastasis. The research of hypoxia in the solid tumor microenvironment is clear, and if a regulatable promoter that is activated under a hypoxia condition can be designed, an expression of downstream protein can be specifically activated in the tumor microenvironment, so as to improve the effectiveness and safety of CAR-T cells.

Although much theoretical research has been conducted on tumor tissue hypoxia, how to apply hypoxic environments in combination with CAR-T therapy still remains a new research direction, requiring extensive research and experimentation.

### Summary of the Present Disclosure

In view of the above, the present disclosure aims to provide a promoter. The promoter can enhance an expression of a target gene under a hypoxic environment, enhance an expression of a target factor (protein, nucleic acid, and the like), and improve efficacy of anti-tumor drugs.

The present disclosure starts from a hypoxic tumor microenvironment, and regulates specific activation of CAR-T by constructing a hypoxia-activated regulatory element to specifically regulate an expression of a protein. Successful construction of the hypoxic environment by in vitro drugs and subsequent CAR-T killing results show that CAR-T under hypoxic conditions induced by CoCl₂ has a stronger killing effect than non-induced CAR-T, indicating that the constructed CAR and CAR-T have the characteristics of passive targeting ability under the hypoxic microenvironment, showing stronger activity and a stronger killing capacity under the hypoxic microenvironment, and exhibit safety characteristics under a non-hypoxic microenvironment. This has important guiding significance for clinical application of CAR-T and development of new strategies for tumor combination therapy.

In order to achieve the foregoing objective, the present disclosure adopts the following technical solutions:
The present disclosure provides a hypoxia-regulatable promoter composed of a Hif1α regulatory element and a mini promoter that are linked to each other. A quantity of repeats in the Hif1α regulatory element is 3 to 5.

Specifically, the Hif1α regulatory element is a tandem of 3 to 5 repeats. The tandem not only refers to direct connection of two nucleotide sequences (that is, the 3' end of one nucleotide sequence and the 5' end of the other nucleotide sequence are directly connected), but also refers to indirect connection of two nucleotide sequences (that is, other nucleotide sequences may be connected between the two nucleotide sequences, as long as they do not affect an expression of functions of the two nucleotide sequences).

Preferably, the quantity of repeats in the Hif1α regulatory element is 3, a nucleotide sequence of the Hif1α regulatory element is set forth in SEQ ID NO: 1, and the promoter is preferably a 3HRE-CMVmini promoter. Further, a structure of the Hif1α regulatory element is optimized to obtain an optimized Hif1α regulatory element, a nucleotide sequence of the Hif1α regulatory element is set forth in SEQ ID NO: 2, and the Hif1α regulatory element can be adapted to different expression vectors after the optimization. Further, a nucleotide sequence of the promoter is set forth in SEQ ID NO: 3.

Preferably, the quantity of repeats in the Hif1α regulatory element is 4, a nucleotide sequence of the Hif1α regulatory element is set forth in SEQ ID NO: 4, and the promoter is preferably a 4HRE-CMVmini promoter. Further, a structure of the Hif1α regulatory element may be optimized to obtain an optimized Hif1α regulatory element, a nucleotide sequence of the Hif1α regulatory element is set forth in SEQ ID NO: 5, and the Hif1α regulatory element can be adapted to different expression vectors after the optimization. Further, a nucleotide sequence of the promoter is set forth in SEQ ID NO: 6.

Preferably, the quantity of repeats in the Hif1α regulatory element is 5, a nucleotide sequence of the promoter is set forth in SEQ ID NO: 7, and the promoter is preferably a 5HRE-CMVmini promoter. Further, two ends of the SEQ ID NO: 7 are optimized to facilitate matching between the promoter and the vector, and a promoter having a nucleotide sequence set forth in SEQ ID NO: 8 is obtained after the optimization.

Further, the promoter is regulated by a hypoxic environment, and the mini promoter is selected from any one of a cell virus promoter, an HSV thymidine kinase promoter, a simian virus 40 promoter, an adenovirus late promoter, and a synthetic promoter. Preferably, the mini promoter is miniCMV.

The present disclosure further provides a CAR construct containing the foregoing promoter.

Specifically, the CAR construct may be a conventional first-generation, second-generation, or third-generation CAR construct, or a new type of CAR construct such as an improved dual CAR construct or a regulatable CAR construct (such as FRB/FKBP12 regulation).

Further, an antigen recognized by an antigen recognition domain of the CAR construct is one or more of CD19, CD20, CD123, CD22, BCMA, ROR1, mesothelin, PSCA, PSMA, c-Met, GPC-3, Her2, EGFRvIII, GD-2, NY-ESO-1TCR, and MAGEA3TCR.

Further, an antigen recognition region of the CAR construct is ScFv, and an amino acid sequence of the ScFv is set forth in SEQ ID NO: 9 or SEQ ID NO: 10 or SEQ ID NO: 11 or SEQ ID NO: 12 or SEQ ID NO: 13 or a functional variant thereof, or the ScFv contains a heavy chain variable region having an amino acid sequence set forth in SEQ ID NO: 14 and a light chain variable region having an amino acid sequence set forth in SEQ ID NO: 15, or contains a heavy chain variable region having an amino acid sequence set forth in SEQ ID NO: 16 and a light chain variable region having an amino acid sequence set forth in SEQ ID NO: 17, or contains a heavy chain variable region having an amino acid sequence set forth in SEQ ID NO: 18 and a light chain variable region having an amino acid sequence set forth in SEQ ID NO: 19.

Further, the CDR1, CDR2, and CDR3 of the heavy chain amino acid sequence set forth in SEQ ID NO: 14 and the light chain amino acid sequence set forth in SEQ ID NO: 15 are at positions 26-33, 51-58, and 97-101 of SEQ ID NO: 14; and at positions 23-31, 49-51, and 88-96 of SEQ ID NO: 15, respectively.

Further, the CDR1, CDR2, and CDR3 of the heavy chain amino acid sequence set forth in SEQ ID NO: 16 and the light chain amino acid sequence set forth in SEQ ID NO: 17 are at positions 26-33, 51-57, and 96-109 of SEQ ID NO: 16; and at positions 27-32, 50-52, and 89-97 of SEQ ID NO: 17.

Further, a hinge region sequence in the CAR construct may be derived from: IgG, CD8, CD7, CD4; a transmembrane region in the CAR construct may be derived from CD8, CD28, CD3ε, CD4, CD16, CD137, CD80, and CD86; an intracellular signal region in the CAR construct can be derived from CD3, CD137, CD28, CD27, OX40, ICOS, GITR, CD2, CD40, PD-1, PD1L, B7-H3, Lymphocyte Function-Associated Antigen 1 (LFA-1), ICAM-1, CD7, NKG2C, CD83, CD86, and CD127.

Preferably, an amino acid sequence of a hinge region of the CAR construct is set forth in SEQ ID NO: 20 or SEQ ID NO: 21 or SEQ ID NO: 22 or SEQ ID NO: 23 or SEQ ID NO: 24 or SEQ ID NO: 25 or a functional variant thereof.

Further, the transmembrane region in the CAR construct can be derived from CD8, CD28, CD3ε, CD4, CD16, CD137, CD80, and CD86.

Preferably, an amino acid sequence of the transmembrane region of the CAR construct is set forth in SEQ ID NO: 26 or SEQ ID NO: 27 or a functional variant thereof.

Further, the intracellular signal region in the CAR construct can be derived from CD3, CD137, CD28, CD27, OX40, ICOS, GITR, CD2, CD40, PD-1, PD1L, B7-H3, Lymphocyte Function-Associated Antigen 1 (LFA-1), ICAM-1, CD7, NKG2C, CD83, CD86, and CD127. The intracellular signal region comprises an intracellular costimulatory domain and an intracellular activation signal.

Preferably, an amino acid sequence of the intracellular costimulatory domain of the CAR construct is set forth in SEQ ID NO: 28 or SEQ ID NO: 29 or SEQ ID NO: 30 or SEQ ID NO: 31 or a functional variant thereof.

Preferably, an amino acid sequence of the intracellular activation signal of the CAR construct is set forth in SEQ ID NO: 32 or a functional variant thereof.

Further, an amino acid sequence of the CAR construct is set forth in SEQ ID NO: 33 or SEQ ID NO: 34 or SEQ ID NO: 35 or SEQ ID NO: 36 or a functional variant thereof.

For a specific amino acid sequence, the "functional variant" thereof generally refers to an amino acid sequence that has substantially the same function (for example, properties of a chimeric antigen receptor) and that has at least 85% (for example, at least 85%, at least 90%, at least 91%, at least 92%, at least 93%, at least 94%, at least 95%, at least 96%, at least 97%, at least 98%, at least 99%, or at least 100%) sequence identity. In some embodiments, the variant of the amino acid sequence has substantially the same function.

In some embodiments, the CAR construct containing the hypoxic promoter has a truncated EGFRt regulatory tag. In some embodiments, the CAR containing the hypoxic promoter is a universal CAR construct. In some embodiments, the CAR construct containing the hypoxic promoter carries a suicide gene such as iCasp9.

In some embodiments, the antigen recognition region may be a ligand/receptor that recognizes a target antigen. In some embodiments, the ScFv may be a ScFv of a murine antibody or a fully human antibody or a human-mouse chimeric antibody, or a single domain antibody such as shark, alpaca or camel antibody, or a bispecific antibody, or a combination of artificially designed target-specific fibronectin type III (FN3) domains that recognize a specific target.

In some embodiments, the CAR construct comprises one or more components of a natural killer cell receptor (NKR), thereby forming an NKR-CAR. The NKR component can be a transmembrane domain, hinge domain, or cytoplasmic domain from any of the following natural killer cell receptors: killer cell immunoglobulin-like receptor (KIR), such as KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1, and KIR3DP1; natural cytotoxic receptors (NCR), such as NKp30, NKp44, NKp46; signaling lymphocytic activation molecule (SLAM) family of immune cell receptors, such as CD48, CD229, 2B4, CD84, NTB-A, CRA, BLAME and CD2F-10; Fc receptor (FcR), such as CD16, and CD64; and Ly49 receptor, such as LY49A and LY49C. The NKR-CAR molecule can interact with an adapter molecule or an intracellular signal domain (such as DAP12).

The present disclosure further aims to provide an isolated nucleic acid molecule that encodes any of the foregoing CAR constructs.

The present disclosure further aims to provide a CAR expression vector containing the promoter according to any one of claims 1 to 11.

Further, a nucleotide sequence of the hypoxic promoter is set forth in SEQ ID NO: 1 or SEQ ID NO: 2 or SEQ ID NO: 3 or SEQ ID NO: 4 or SEQ ID NO: 5 or SEQ ID NO: 6 or SEQ ID NO: 7 or SEQ ID NO: 8. The expression vector may be any one of a lentivirus expression vector, a retrovirus expression vector, an adenovirus expression vector, an adeno-associated virus expression vector, a DNA vector, an RNA vector, and a plasmid.

Preferably, the vector is a lentiviral vector.

In some embodiments, the lentiviral vector is selected from a group including essentially of: Human Immunodeficiency Virus 1 (HIV-1), Human Immunodeficiency Virus 2 (HIV-2), Visna-midi Virus (VMV) virus, Caprine Arthritis-encephalitis Virus (CAEV), Equine Infectious Anemia Virus (EIAV), Feline Immunodeficiency Virus (FIV), Bovine Immunodeficiency Virus (BIV), and Simian Immunodeficiency Virus (SIV).

In some embodiments, the vector contains a left (5') retroviral LTR, a Psi (Ψ) packaging signal, a central polypurine tract/DNA FLAP (cPPT/FLAP), a retroviral export element, a promoter operably linked to a polynucleotide encoding a CAR encompassed herein, and a right (3') retroviral LTR.

In some embodiments, the CAR containing a hepatitis B virus post-transcriptional regulatory element (HPRE) or woodchuck post-transcriptional regulatory element (WPRE), and an optimized woodchuck post-transcriptional regulatory element (oPRE).

In some embodiments, the promoter of the 5' LTR is replaced with a heterologous promoter.

In some embodiments, the heterologous promoter is a Cytomegalovirus (CMV) promoter, a Rous Sarcoma Virus (RSV) promoter, or a simian Virus 40 (SV40) promoter.

In some embodiments, the 5 'LTR or 3' LTR is a lentiviral LTR.

In some embodiments, the 3' LTR is a self-inactivating (SIN) LTR.

In some embodiments, a polynucleotide encoding a CAR encompassed herein contains an optimized Kozark sequence.

In some embodiments, the promoter operably linked to a polynucleotide encoding a CAR encompassed herein is selected from the group including: a cytomegalovirus immediate early gene promoter (CMV), an elongation factor 1 α promoter (EF1- α), a phosphoglycerate kinase-1 promoter (PGK), a ubiquitin-C promoter (UBQ-C), a cytomegalovirus enhancer/chicken β-actin promoter (CAG), a polyoma enhancer/herpes simplex thymidine kinase promoter (MC1), a β-actin promoter (β-ACT), a simian virus 40 promoter (SV40), a myeloproliferative sarcoma virus enhancer, and a (MND) promoter with negative control region deleted and d1587rev primer binding site substituted.

**Table 1 Nucleotide sequences corresponding to promoters**

| | |
|---|---|
| WTPGK promoter | SEQ ID NO:48 |
| Truncated PGK promoter 1 | SEQ ID NO:49 |
| Truncated PGK promoter 2 | SEQ ID NO:50 |
| Truncated PGK promoter 3 | SEQ ID NO:51 |
| Truncated PGK promoter 4 | SEQ ID NO:52 |
| EFla promoter | SEQ ID NO:53 |
| MND promoter | SEQ ID NO:54 |

In some embodiments, the vector containing CAR may contain a secreted anti-PD-lScFv. In some embodiments, the vector containing CAR contains a PD-1 conjugated transduction peptide (such as PD-1-CD28-CD137-CD3 signaling construct). In some embodiments, the vector containing CAR contains multiple CAR combinations, such as 2 CAR combinations targeting different antigens or different recognition sites of the same antigen.

Preferably, a nucleotide sequence of the vector is set forth in SEQ ID NO: 37 or SEQ ID NO: 38 or SEQ ID NO: 39 or SEQ ID NO: 40 or SEQ ID NO: 41 or SEQ ID NO: 42 or SEQ ID NO: 43 or SEQ ID NO: 44.

Preferably, when the quantity of repeats in the Hif1α regulatory element is 5, the expression vector is _{P}BKL1-5H1P-CAR/anti-PSCA-oPRE, which contains the CAR construct with a nucleotide sequence set forth in SEQ ID NO: 45; the vector is pBKL1-5H1P-CAR/anti-CD19-oPRE, which contains the CAR construct having a nucleotide sequence set forth in SEQ ID NO: 46; the vector is pBKL1-5H1P-CAR/anti-CEA-oPRE, which contains the CAR construct having a nucleotide sequence set forth in SEQ ID NO: 47.

The present disclosure further provides a CAR-T cell containing any of the forgoing expression vectors. The CAR-T cell can induce activation under a hypoxic environment in the presence of a target antigen. The expression capacity and abundance of CAR after stimulation of target antigen under a hypoxic environment are improved, which enhances the effectiveness of CAR-T. The expression capacity and abundance of CAR after stimulation of target antigen under a non-hypoxic environment are lower than conventional CAR-T, and the safety is higher.

The present disclosure further provides a method for improving a killing capacity of a CAR-T cell under a hypoxic environment, which is characterized by constructing an expression vector of any of the forgoing CAR constructs and infecting T lymphocytes, and then acting on the target cell.

Specifically, it is to construct a CAR construct containing a hypoxia-regulatable promoter. The hypoxia-regulatable promoter is composed of a Hif1α regulatory element and a mini promoter that are linked to each other. The quantity of repeats in the Hif1α regulatory element is 3 to 5. The mini promoter is selected from a cell virus promoter, an HSV thymidine kinase promoter, a simian virus 40 promoter, an adenovirus late promoter or a synthetic promoter.

Preferably, the mini promoter is miniCMV.

Preferably, the quantity of repeats in the Hif1α regulatory element is 3, a nucleotide sequence of the Hif1α regulatory element is set forth in SEQ ID NO: 1, and the promoter is preferably a 3HRE-CMVmini promoter. Further, a structure of the Hif1α regulatory element is optimized to obtain an optimized Hif1α regulatory element, a nucleotide sequence of which is set forth in SEQ ID NO: 2, and the Hif1α regulatory element can be adapted to different expression vectors after optimization. Further, a nucleotide sequence of the promoter is set forth in SEQ ID NO: 3.

Preferably, the quantity of repeats in the Hif1α regulatory element is 4, a nucleotide sequence of the Hif1α regulatory element is set forth in SEQ ID NO: 4, and the promoter is preferably a 4HRE-CMVmini promoter. Further, a structure of the Hif1α regulatory element is optimized to obtain an optimized Hif1α regulatory element, a nucleotide sequence of which is set forth in SEQ ID NO: 5, and the Hif1α regulatory element can be adapted to different expression vectors after the optimization. Further, a nucleotide sequence of the promoter is set forth in SEQ ID NO: 6.

Preferably, the quantity of repeats in the Hif1α regulatory element is 5, a nucleotide sequence of the promoter is set forth in SEQ ID NO: 7, and the promoter is preferably a 5HRE-CMVmini promoter. Further, two ends of the SEQ ID NO: 7 are optimized to facilitate the matching of the promoter and the vector, and a promoter having a nucleotide sequence set forth in SEQ ID NO: 8 is obtained after the optimization.

The present disclosure further provides a method for improving an ability of a CAR-T cell to secrete IFN-γ and/or IL-2 factors under a hypoxic environment, which is characterized by constructing a CAR construct containing a hypoxia-regulatable promoter. The hypoxia-regulatable promoter is composed of a Hif1α regulatory element and a mini promoter that are linked to each other. The quantity of repeats in the Hif1α regulatory element is 3 to 5. The mini promoter is selected from a cell virus promoter, an HSV thymidine kinase promoter, a simian virus 40 promoter, an adenovirus late promoter or a synthetic promoter.

Specifically, the present disclosure constructs a CAR construct containing a hypoxia-regulatable promoter. The hypoxia-regulatable promoter is composed of a Hif1α regulatory element and a mini promoter that are linked to each other. The quantity of repeats in the Hif1α regulatory element is 3 to 5. The mini promoter is selected from a cell virus promoter, an HSV thymidine kinase promoter, a simian virus 40 promoter, an adenovirus late promoter or a synthetic promoter.

Preferably, the mini promoter is miniCMV.

Preferably, the quantity of repeats in the Hif1α regulatory element is 3, a nucleotide sequence of the Hif1α regulatory element is set forth in SEQ ID NO: 1, and the promoter is preferably a 3HRE-CMVmini promoter. Further, a structure of the Hif1α regulatory element is optimized to obtain an optimized Hif1α regulatory element, a nucleotide sequence of which is set forth in SEQ ID NO: 2, and the Hif1α regulatory element can be adapted to different expression vectors after the optimization. Further, a nucleotide sequence of the promoter is set forth in SEQ ID NO: 3.

Preferably, the quantity of repeats in the Hif1α regulatory element is 4, a nucleotide sequence of the Hif1α regulatory element is set forth in SEQ ID NO: 4, and the promoter is preferably a 4HRE-CMVmini promoter. Further, a structure of the Hif1α regulatory element is optimized to obtain an optimized Hif1α regulatory element, a nucleotide sequence of which is set forth in SEQ ID NO: 5, and the Hif1α regulatory element can be adapted to different expression vectors after the optimization. Further, a nucleotide sequence of the promoter is set forth in SEQ ID NO: 6.

Preferably, the quantity of repeats in the Hif1α regulatory element is 5, a nucleotide sequence of the promoter is set forth in SEQ ID NO: 7, and the promoter is preferably a 5HRE-CMVmini promoter. Further, two ends of the SEQ ID NO: 7 are optimized to facilitate the matching between the promoter and the vector, and a promoter having a nucleotide sequence set forth in SEQ ID NO: 8 is obtained after optimization.

The present disclosure further provides application of the forgoing promoter or the forgoing CAR construct or the forgoing nucleic acid molecule or the forgoing expression vector or the forgoing CAR-T cell in preparation of tumor drugs.

Further, the tumor comprise acute lymphoid leukemia, chronic lymphocytic leukemia, chronic myelogenous leukemia, non-Hodgkin's lymphoma, Hodgkin's lymphoma, prostate cancer, colorectal cancer, breast cancer, ovarian cancer, cervical cancer, membranous adenocarcinoma, lung cancer, kidney cancer, liver cancer, brain cancer and skin cancer; and the tumor highly expresses one or more of CD19, CD20, CD123, CD22, BCMA, R0R1, CEA, mesothelin, PSCA, PSMA, c-Met, GPC- 3, Her2, EGFRvIII, GD-2, NY-ESO-1TCR, and MAGE A3 TCR.

Specifically, the cells can be used in combination with other active agents and/or treatments that can enhance CAR expression activity.

Specifically, the active agent and/or treatment may be surgery, chemotherapy, radiation, immunosuppressive agents, such as cyclosporin, azathioprine, methotrexate, mycophenolate and FK506, antibodies or other immunoablative agents, such as CAMPATH, anti-CD3 antibodies or other antibody therapy, cytoxan, fludarabine, cyclosporin, FK506, rapamycin, mycophenolic acid, steroids, FR901228, cytokines and radiation.

In some embodiments, the cells may express other active agents, for example, active agents that enhance the activity of CAR expressing cells. The active agent may be an active agent that blocks inhibitory molecules. Inhibitory molecules such as PD1 can reduce an ability of a CAR-expressing cell to drive immune effector responses in some implementations. Inhibitory molecules includes PD1, PD-L1, CTLA4, TIM3, LAG3, VISTA, BTLA, TIGIT, LAIR1, CD160, 2B4, CEACAM (CEACAM-1, CEACAM-3, CEACAM-5), LAG3, VISTA, BTLA, TIG, LAIR1, CD160, 2B4, CD80, CD86, B7-H3 (CD276), B7-H4 (VTCN1), HVEM (TNFRSF14 or CD270), KIR, A2aR, MHC type I, MHC type II, GAL9, adenosine, TGFR (TGFRβ) and TGFRβ. The extracellular domain (amino acid sequence set forth in SEQ ID NO: 55) of the inhibitory molecule can be fused to the transmembrane domain and intracellular signaling domain, such as PD1 CAR (nucleotide sequence set forth in SEQ ID NO: 56 or SEQ ID NO: 57).

### Beneficial effects of the present disclosure are as follows.

The present disclosure provides use of a hypoxia-regulatable promoter in CAR-T, which utilizes the fact that the promoter induced by the hypoxia microenvironment can enhance the expression of target genes, proteins and other factors under a hypoxic environment, and improve the therapeutic effect of tumor drugs and the effectiveness and safety of CAR-T therapy.

The CAR construct induced by the hypoxic microenvironment provided by the present disclosure can not only be effectively expressed on T lymphocytes, but also can enhance the activation of CAR-T cells under a hypoxic environment, enhance the effectiveness of CAR-T, and improve an ability of a CAR-T cell to secrete IFN-γ and IL-2 factor, improve the killing of CAR-T cells against tumor target cells, and can be used for targeted therapy of tumors.

The expression capacity and abundance of CAR-T cells induced by the hypoxic microenvironment provided by the present disclosure after stimulation of target antigen under a non-hypoxic environment are lower than conventional CAR-T, which improves safety.

The CAR-T cells provided by the present disclosure has enhanced activity under a hypoxic environment, which can be used for the preparation of adoptive cell therapy in tumor drugs, and has important guiding significance in the clinical application of CAR-T and the development of new strategies for tumor combined therapy.

### Brief Description of the Drawings

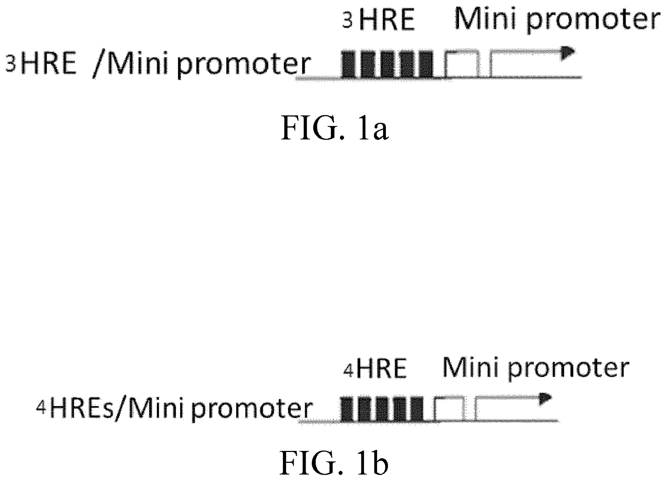

Fig. 1a is a structural diagram of a 3H1P promoter.
Fig. 1bis a structural diagram of a 4H1P promoter.
Fig. 1c is a structural diagram of a pBKL1-5H1P-CAR vector.
Fig. 1d is a structural diagram of a pBKL1-5H1P-CAR vector.
Fig. 2a shows the verification of a hypoxia induction scheme for the 3H1P promoter.

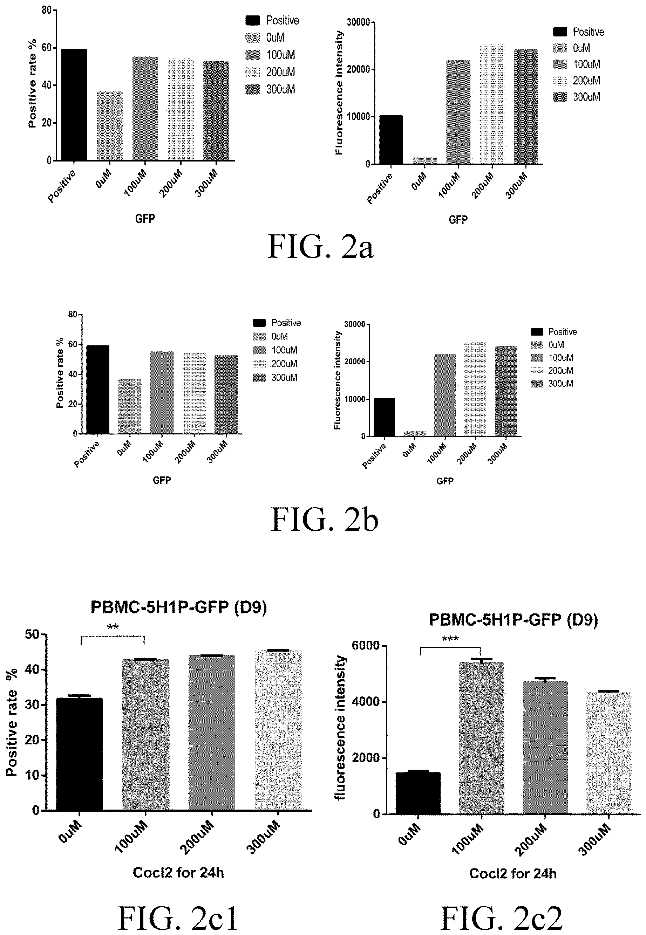

Fig. 2a shows the verification of a hypoxia induction scheme for the 4H1P promoter.
Fig. 2c1 is a histogram of the GFP positive rate verified by a hypoxia induction scheme for a 5H1P promoter, and Fig. 2c2 is a histogram of the fluorescence density of GFP expression verified by the hypoxia induction scheme for the 5H1P promoter.

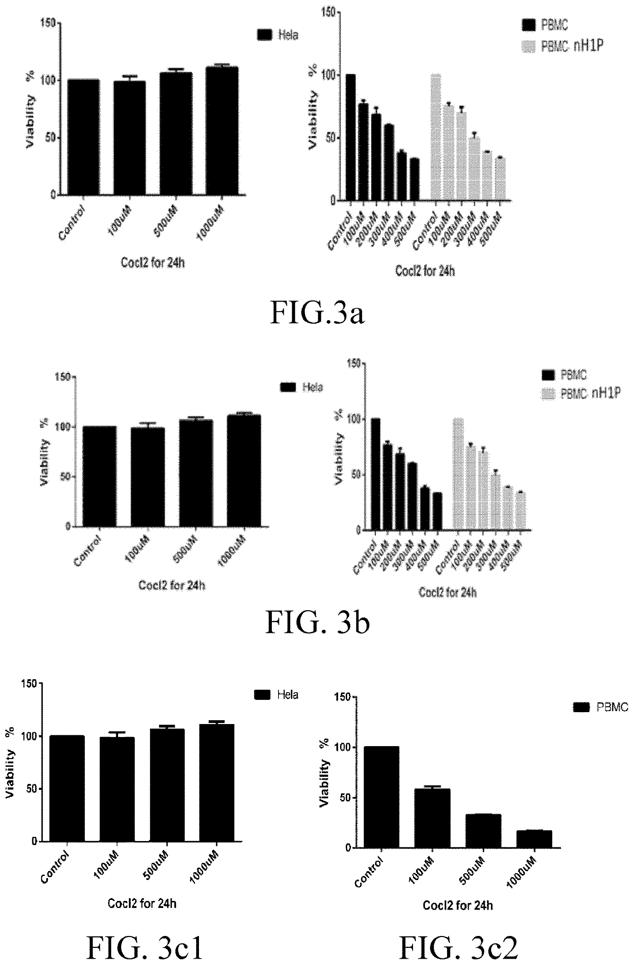

Fig. 3a shows the effect of 3H1P promoter hypoxia-induced drugs on cell proliferation.
Fig. 3b shows the effect of 4H1P promoter hypoxia-induced drugs on cell proliferation.
Fig. 3c1 is a histogram showing the effect of 5H1P promoter hypoxia-induced drugs on cell proliferation (Hela), and Fig. 3c2 is a histogram showing the effect of 5H1P promoter hypoxia-induced drugs on cell proliferation (PBMC).

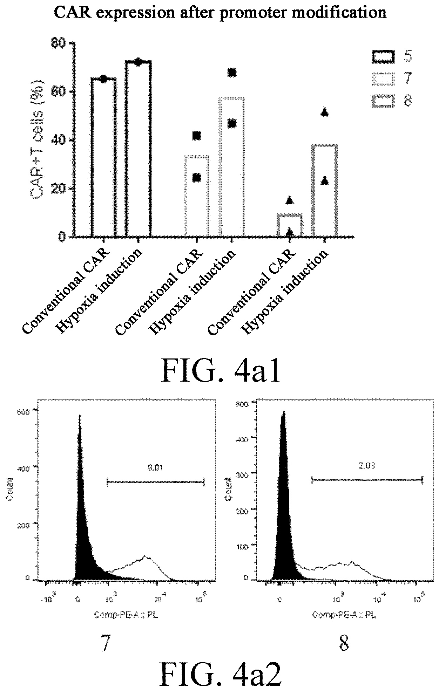

Fig. 4a1 shows the detection of CAR positive rate for a 3H1P promoter, and Fig. 4a2 shows the detection of CAR expression intensity for the 3H1P promoter after hypoxia induction.

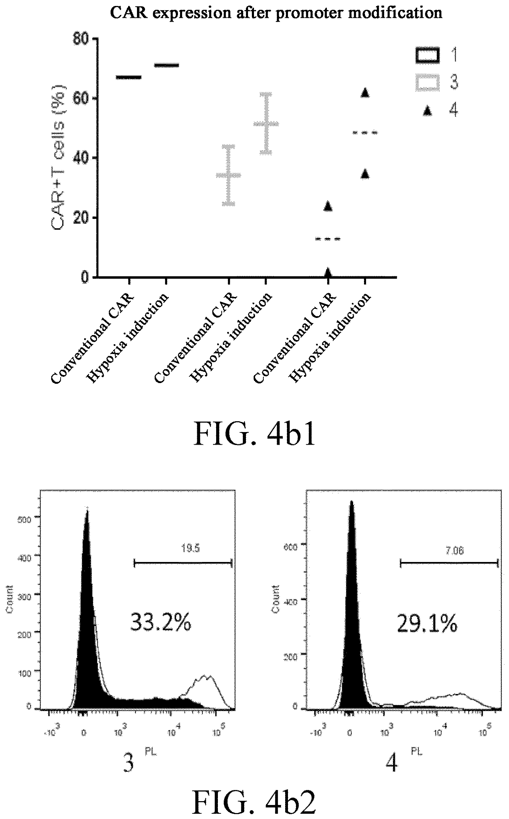

Fig. 4b1 shows the detection of CAR positive rate for a 4H1P promoter, and Fig. 4b2 shows the detection of CAR expression intensity for the 4H1P promoter after hypoxia induction.

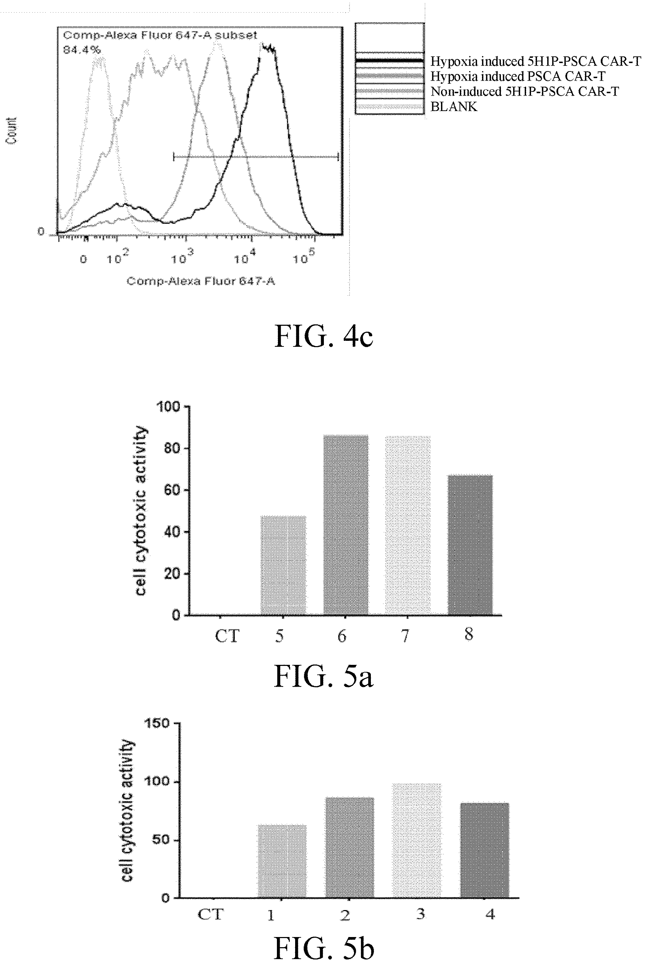

Fig. 4c shows CAR positive rate for a 5H1P promoter detected by flow cytometry.
Fig. 5a shows the validation of effectiveness of CAR-T modified by the 3H1P promoter.
Fig. 5b shows the validation of effectiveness of CAR-T modified by the 4H1P promoter.

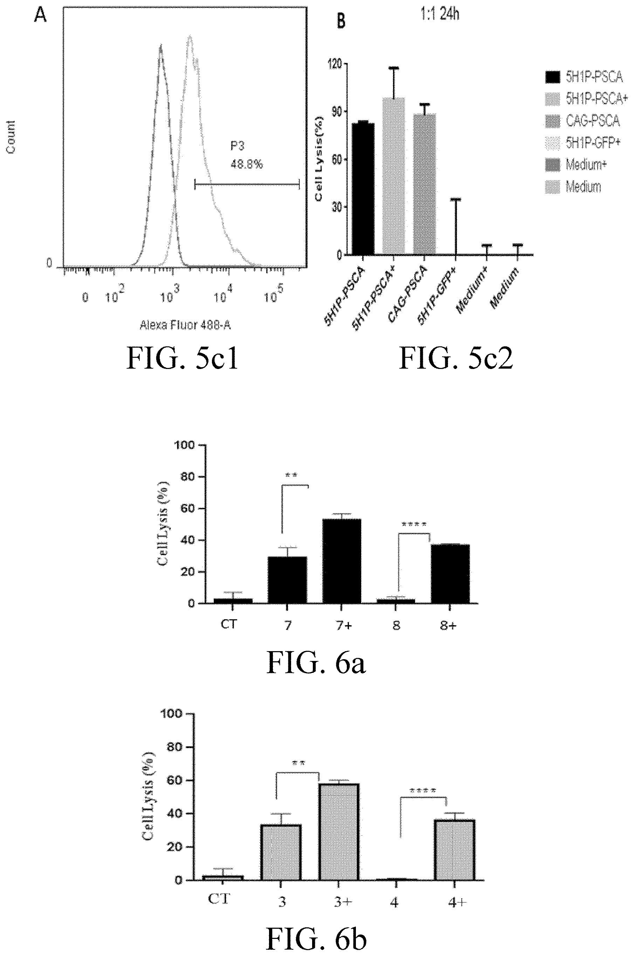

Fig. 5c1 shows the PSCA expression of a target cell Hela; and Fig. 5c2 shows the killing efficiency of CAR-T modified by the 5H1P promoter on the target cell Hela.
Fig. 6a shows the comparison of activity of CAR-T modified by the 3H1P promoter under hypoxic and non-hypoxic environments.
Fig. 6b shows the comparison of activity of CAR-T modified by the 4H1P promoter under hypoxic and non-hypoxic environments.

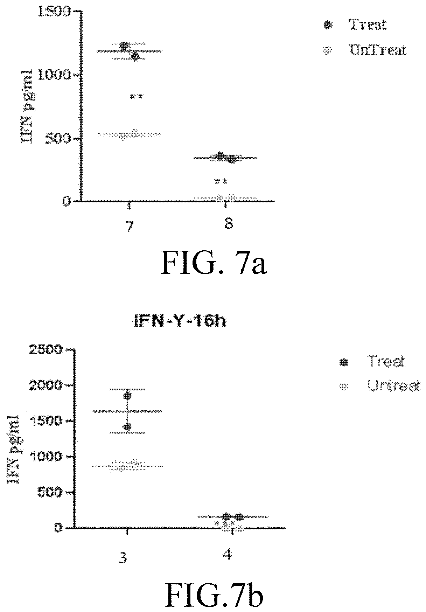

Fig. 7a shows the comparison of factor secretion of CAR-T activity after 3H1P promoter modification under hypoxic and non-hypoxic environments.
Fig. 7b shows the comparison of factor secretion of CAR-T activity after 4H1P promoter modification under hypoxic and non-hypoxic environments.

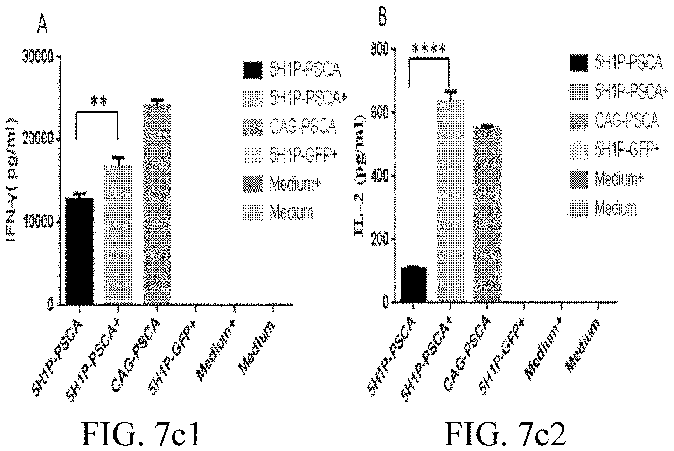

Fig. 7c1 shows the detection of IFN-γ factor secretion ability after CAR-T activation, and Fig. 7c2 shows the detection of IL-2 factor secretion ability after CAR-T activation.

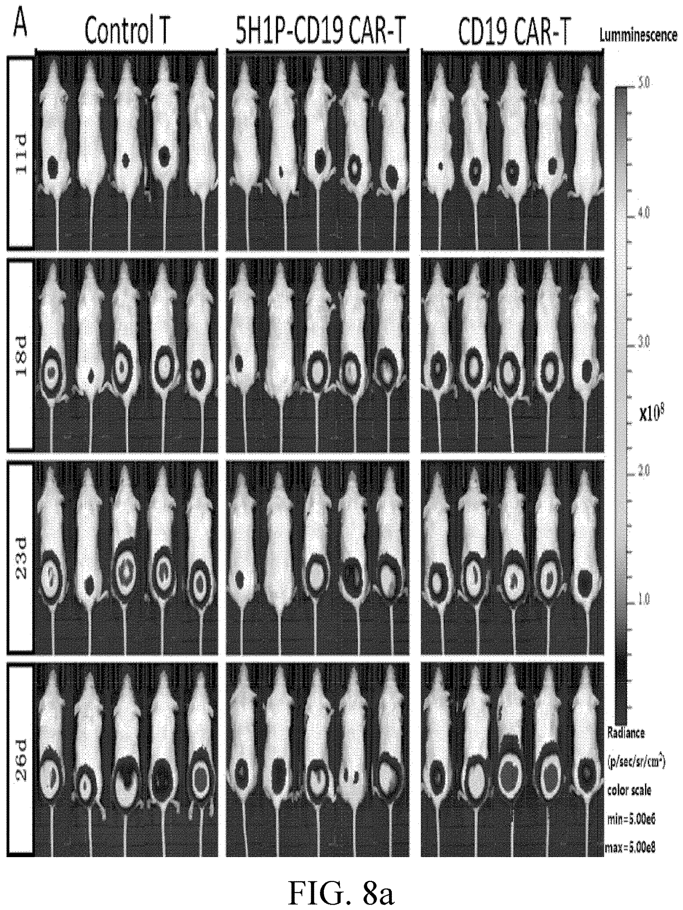

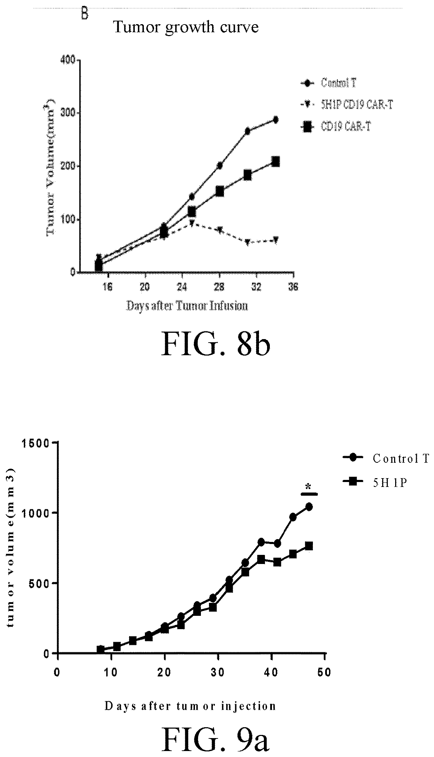

Fig. 8a shows the validation of effectiveness of CAR-T in lymphoma tumor-bearing mice after 5H1P hypoxic promoter modification *in vivo,* and Fig. 8b is a diagram showing the corresponding tumor growth curve.
Fig. 9a shows the inhibition of CAR-T in tumor proliferation in human colorectal cancer tumor-bearing mice after 5H1P hypoxic promoter modification *in vivo.*

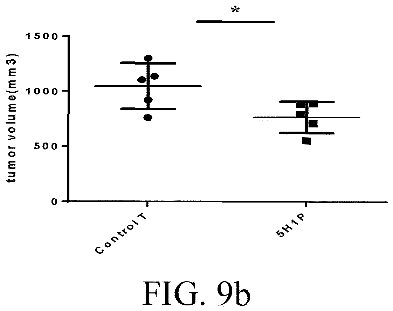

Fig. 9b shows the effect of CAR-T on the tumor volume in human colorectal cancer tumor-bearing mice after 5H1P hypoxic promoter modification *in vivo.*

### Detailed Description of the Preferred Embodiments

Hereinafter, the preferred embodiments of the present disclosure will be described in detail with reference to the accompanying drawings. The experimental methods without specified conditions in the preferred embodiments usually follow conventional conditions, such as the conditions described in the Molecular Cloning: A Laboratory Manual (third edition, J. Sambrook et al.), or the conditions recommended by the manufacturer. The embodiments provided are to better describe the content of the present disclosure, which does not mean that the content of the present disclosure is only limited thereto. Therefore, the non-essential improvements and adjustments to the embodiments by those skilled in the art based on the above summary of the disclosure still belong to the protection scope of the present disclosure.

### Embodiment 1 Plasmid construction

### (1) Construction of a promoter "3HRE-CMVmini promoter-NheI (3H1P)" that matches the lentiviral vector

A promoter "3HRE-CMVmini promoter-NheI (3H1P)" that matches the lentiviral vector was constructed, having a nucleotide sequence set forth in SEQ ID NO: 3, which was cut by double digests respectively and recovered to obtain fragments. The gene fragments were ligated and transformed, and single clones were picked to obtain recombinant vectors. The vectors were numbered as 5, 6, 7, and 8 respectively. The original CAR construct contains: ScFv targeting PSCA or CEA and having an amino acid sequence set forth in SEQ ID NO: 9 or SEQ ID NO: 10; a hinge construct having an amino acid sequence set forth in SEQ ID NO: 20, SEQ ID NO: 21 or SEQ ID NO: 22 or SEQ ID NO: 23 or SEQ ID NO: 24 or SEQ ID NO: 25; a transmembrane construct having an amino acid sequence set forth in SEQ ID NO: 26 or SEQ ID NO: 27, an intracellular costimulatory domain having an amino acid sequence set forth in SEQ ID NO: 28 or SEQ ID NO: 29 or SEQ ID NO: 30 or SEQ ID NO: 31, and an intracellular activation signal having an amino acid sequence set forth in SEQ ID NO: 32; a nucleotide sequence of the obtained CAR modified by the hypoxic promoter is set forth in SEQ ID NO: 37, SEQ ID NO: 38, SEQ ID NO: 39 and SEQ ID NO: 40, and a CAR amino acid sequence is set forth in SEQ ID NO: 33, SEQ ID NO: 34, SEQ ID NO: 35 and SEQ ID NO: 36. Fig. 1a is a structural diagram of the promoter 3HRE-CMVmini promoter-NheI.

### (2) Construction of a promoter "4HRE-CMVmini promoter-NheI (4H1P)" that matches the lentiviral vector

A promoter "4HRE-CMVmini promoter-NheI (4H1P)" that matches the lentiviral vector was constructed, having a nucleotide sequence set forth in SEQ ID NO: 6, which was cut by double digests respectively and recovered to obtain fragments, the gene fragments were ligated and transformed, and single clones were picked to obtain recombinant vectors. The vectors were numbered as 1, 2, 3, and 4 respectively. The original CAR construct contains: ScFv targeting PSCA or CEA having an amino acid sequence set forth in SEQ ID NO: 9 or SEQ ID NO: 10; a hinge construct having an amino acid sequence set forth in SEQ ID NO: 20, SEQ ID NO: 21 or SEQ ID NO: 22 or SEQ ID NO: 23 or SEQ ID NO: 24 or SEQ ID NO: 25; a transmembrane construct having an amino acid sequence set forth in SEQ ID NO: 26 or SEQ ID NO: 27, an intracellular costimulatory domain having an amino acid sequence set forth in SEQ ID NO: 28 or SEQ ID NO: 29 or SEQ ID NO: 30 or SEQ ID NO: 31, and an intracellular activation signal having an amino acid sequence set forth in SEQ ID NO: 32; the nucleotide sequence of the obtained CAR modified by the hypoxic promoter is set forth in SEQ ID NO: 41, SEQ ID NO: 42, SEQ ID NO: 43 and SEQ ID NO: 44, and a CAR amino acid sequence is set forth in SEQ ID NO: 33, SEQ ID NO: 34, SEQ ID NO: 35 and SEQ ID NO: 36. Fig. 1b is a structural diagram of the promoter 4HRE-CMVmini promoter-NheI.

### (3) Construction of a promoter "5HRE-CMVmini promoter-NheI (5H1P)" that matches the lentiviral vector

Plasmids were constructed according to the conditions shown in Table 2 for subsequent experiments, taking mini promoter "miniCMV", lentivirus expression vector, antigen recognition region CD19 and PSCA as examples for plasmid construction.

The nucleic acid sequence of the synthetic hypoxic promoter sequence 5HRE-CMVmini promoter was set forth in SEQ ID NO: 7. The promoter EcoRV-5HRE-CMVmini promoter-NheI (5H1P) matched with the lentiviral vector was further constructed, having a nucleic acid sequence set forth in SEQ ID NO. 8. It was cut by double digests and recovered to obtain fragments, the gene fragments were ligated and transformed, and single clones were picked to obtain recombinant vectors PBKL1-5H1P-PSCA (1) -OPRE, PBKL1-5H1P-CD19-OPRE and PBKL1-5H1P-CEA-OPRE, having the nucleotide sequences set forth in SEQ ID NO: 45, SEQ ID NO: 46 and SEQ ID NO: 47 respectively; and PBKL1-5H1P-GFP-OPRE was constructed, which was a control vector that does not contain the CAR gene.

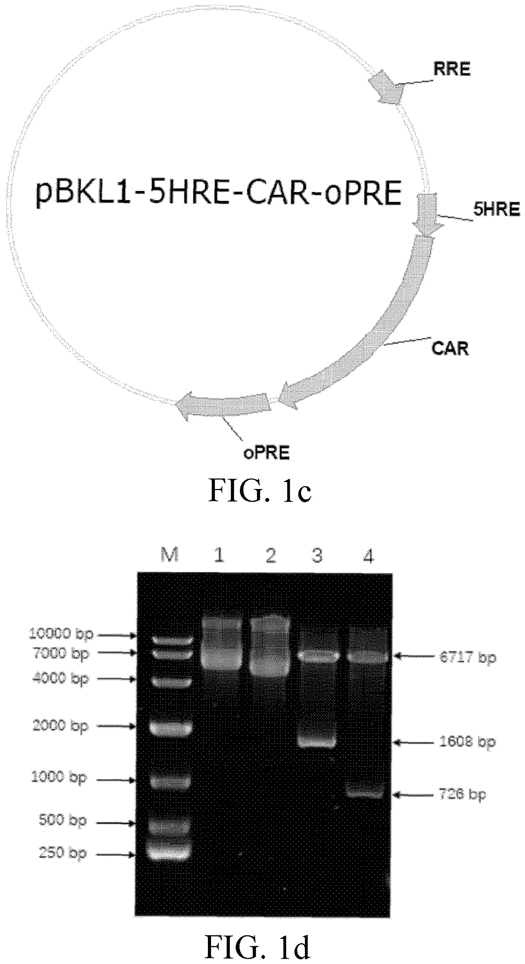

The nucleic acid sequence of the hypoxic promoter and plasmid construct is set forth in SEQ ID NO:58, and Fig. 1c is a diagram showing the CAR construct and a hypoxia inducible vector containing the CAR. The obtained plasmid was verified by double digests Nhel + SalI and verified by sequencing, and the result is shown in Fig. 1d: M: DL10000 DNA molecular weight standard; 1: plasmid PBKL1-5H1P-PSCA (1)-OPRE (8325 bp); 2: plasmid PBKL1-5H1P-GFP-OPRE (7443bp); 3: 6717bp and 1608bp fragments obtained from plasmid PBKL1-5H1P-PSCA (1)-OPRE by Nhel + SalI double digests; 4: 6717bp and 726bp fragments obtained from plasmid PBKL1-5H1P-GFP-OPRE by NheI+SalI double digests.

**Table 2 Conditions for plasmid construction**

| | | | | | |
|---|---|---|---|---|---|
| Mini promoter | Hypoxic promoter sequence | Expression vector | Antigen recognition region | Intracellular domain | CD3 signal transduction domain (SEQ ID NO: 32), CD8 hinge construct (SEQ ID NO: 20), CD7 hinge construct (SEQ ID NO: 21), CD8 mutant hinge construct (SEQ ID NO: 22), IgG4 hinge construct (SEQ ID NO: 23), IgG4 mutant hinge construct (SEQ ID NO: 24), IgD hinge construct (SEQ ID NO: 25), CD28 transmembrane (SEQ ID NO: 26), CD8 transmembrane (SEQ ID NO: 27) |
| miniCMV | 5HRE-CMV mini promoter | Lentivirus expression vector | Any one or more of CD19, CD20, CD123, CD22, BCMA, ROR1, mesothelin, PSCA, PSMA, c-Met, GPC-3, Her2, EGFRvIII, GD-2, NY-ESO-1TCR, MAGE A3TCR | CD28 (SEQ ID NO: 28), 4-1BB (SEQ ID NO: 29), OX40, ICOS (SEQ ID NO: 30), CD27 (SEQ ID NO: 31) | |
| Mini- T K | 5HRE-TKmini promoter | Retrovirus expression vector | | | |
| SV40 | 5HRE-SV40mini promoter | Adenovirus expression vector | | | |
| MLP | 5HRE-MLP promoter | Adeno-associated virus expression vector herpes simplex virus Vector | | | |
| | | DNA vector | | | |
| Synthetic promoter | 5HRE-synthetic promoter | RNA vector | | | |
| | | Plasmid | | | |

### Embodiment 2 Verification of in vitro hypoxia model

The principle of CoCl₂ induced hypoxia: divalent cobalt ions (Co²⁺) in cobalt dichloride (CoCl₂) can replace PHD cofactor divalent iron ions since Co²⁺ has a low affinity to oxygen, which leads to that heme cannot bind to oxygen and to be transformed into an oxidized state, thereby resulting in a hypoxia state.

### (1) Verification of hypoxia induction scheme:

PBMCs infected and activated by PBKL1-3HRE-GFP, PBKL1-4HRE-GFP and PBKL1-5HRE-GFP viruses are respectively used for constructing an in-vitro hypoxia cell model, and the solution was changed after culturing for 12-18 hours. A hypoxic environment was induced by using CoCl₂, after culturing to the 4th day, whether the construction of a hypoxia model was completed was verified through green fluorescence detection and WB. A control group (without adding CoCl₂), an experimental group (adding CoCl₂) and a positive group (normally infected GFP cells without containing a hypoxia-regulatable promoter) were respectively provided, and fluorescence microscopy was carried out to detect the expression intensity of GFP after 24 hours of drug treatment. Also, protein was extracted according to the instructions of M-PER Mammalian Protein Extraction Reagent (Thermo) for detection of the expression of HIF-1a in cells by WB.

As shown in Fig. 2a, it is found that the GFP expression in the CoCl₂-added induction group (that is, hypoxia microenvironment group) is significantly higher than that in the CoCl₂-free group, and the fluorescence intensity is much higher than that in the positive control group, indicating that the hypoxic promoter of PBKL1-3HRE-GFP is able to normally initiate the expression of downstream GFP under the hypoxic environment.

As shown in Fig. 2b, it is found that the GFP expression in the CoCl₂-added induction group (that is, hypoxia microenvironment group) was significantly higher than that in the CoCl₂-free group, and the fluorescence intensity is much higher than that in the positive control group, indicating that the hypoxic promoter of PBKL1-4HRE-GFP is able to normally initiate the expression of downstream GFP under the hypoxic environment.

As shown in Fig. 2c1 and 2c2, it is found that the GFP expression in the CoCl₂-added induction group (that is, hypoxia microenvironment group) is significantly higher than that in the CoCl₂-free group, and the fluorescence intensity is much higher than that in the positive control group, indicating that the hypoxic promoter of PBMC-5H1P-GFP is able to normally initiate the expression of downstream GFP under the hypoxic environment.

### (2) The effect of hypoxia-inducing drugs on cell proliferation

In the morning, an appropriate amount of Hela and TOMC cells were plated on a 96-well plate. After the cells were adhered to the wall for 5 hours, the drugs were added to achieve 0, 100, 200, 300, 400, 500 uM of CoCl₂ final concentration. After treating for 24 hours, CellTiter-Glo One Solution Assay reagent incubated was added in a 1:1 volume, incubated at room temperature for 10 minutes, and then placed in a microplate reader to measure the absorbance.

As shown in Fig. 3a, it is found that the addition of CoCl₂ does not affect the original cell proliferation. Therefore, for PBKL1-3HRE-GFP promoter, the results of CAR-T cell killing will not be affected by the externally added CoCl₂, only relating to whether the hypoxic promoter is contained.

As shown in Fig. 3b, it is found that the addition of CoCl₂ does not affect the original cell proliferation. Therefore, for PBKL1-4HRE-GFP promoter, the results of CAR-T cell killing will not be affected by the externally added CoCl₂, only relating to whether the hypoxic promoter is contained.

As shown in Fig. 3c1 and 3c2, it is found that the addition of CoCl₂ does not affect the original cell proliferation. Therefore, for PBKL1-5HRE-GFP promoter, the results of CAR-T cell killing will not be affected by the externally added CoCl₂, only relating to whether the hypoxic promoter is contained.

### Embodiment 3 Preparation of lentivirus and infection of T lymphocytes

In this embodiment, the 3H1P promoter and the 4H1P and 5H1P promoters were used for experiments, respectively.

A calcium phosphate method was used for the packaging of the lentivirus in this embodiment, which specifically comprises: culturing 293T cells to a good state with DMEM medium containing 10% FBS (w/v), so that the cell confluence was 70-80% upon transfection, and the cell suspension was changed in advance. The packaging plasmid (RRE:REV:2G) and the expression plasmid were added to 1.5 centrifuge tube in a certain ratio, 2.5 mol/L CaCl₂ was added, and ddH₂O was added to the total volume of 600 ul, and mixed evenly. 2 × HBS was added dropwise to the mixture with a pipette, mixed evenly and allowed to stand for 15 min at room temperature, and the mixture was added to a processed 293T cell culture. After 3-5 hours, the medium was replaced again with 10 mL DMEM medium containing 10% FBS. After 48 hours or 72 hours, the cell supernatant was collected, and the virus was purified to for tittering.

Lymphocytes were separated by gradient centrifugation; after centrifugation, the second layer of white lymphocytes was taken, washed with physiological saline, and cultured by adding RPMI 1640 complete medium containing 10% FBS to obtain human PBMC cells. After being activated by anti-CD3 and anti-CD28 monoclonal antibodies for 24 hours, the activated PBMC obtained were infected according to a certain multiplicity of infection (MOI), and polybrene was added to help the virus infection. After culturing overnight, the medium was changed (10% RPMI 1640+IL- 2+ double antibody), continued to culture in a 37°C incubator, and the positive rate of CAR-T was detected on the 12th day of virus infection. A detection method was flow detection. The antibodies were: Protein-L-PE. Protein-L can recognize the light chain of the antibody, and the light chain of the ScFv sequence in the CAR antigen recognition region can be recognized by Protein-L. Therefore, Protein-L can be used to detect the CAR positive rate.

The experimental results of the 3H1P promoter are shown in Fig. 4a1 and 4a2: CAR-T cells with different constructs modified by the 3H1P promoter significantly increase CAR expression under the induced hypoxic environment. Fig. 4a1 shows CAR-T cells with different constructs modified by the 3H1P promoter, the black histogram is the CAR expression intensity of the CAR modified by the promoter under a non-hypoxic environment; and the white histogram is the CAR expression intensity of the CAR modified by the promoter under the hypoxic environment. The expression intensity of CAR is enhanced under the induced hypoxic environment.

The experimental results of the 4H1P promoter are shown in Fig. 4b1 and 4b2: CAR-T cells with different constructs modified by the 4H1P promoter significantly increase CAR expression under the induced hypoxic environment.

Fig. 4b1 shows CAR-T cells with different constructs modified by the 4H1P promoter, the black histogram is the CAR expression intensity of the CAR modified by the promoter under the non-hypoxic environment; and the white histogram is the CAR expression intensity of the CAR modified by the promoter under the hypoxic environment. The expression intensity of CAR is enhanced under the induced hypoxic environment.

The experimental results of the 5H1P promoter are shown in Fig. 4c: the hypoxia-induced 5H1P-PSCA CAR-T group is the CoCl₂-induced CAR-T group with the hypoxic promoter, the hypoxia-induced PSCA CAR-T group is the CoCl₂-induced CAR-T group without the hypoxic promoter, the non-induced 5H1P-PSCA CAR-T group is the non-CoCl₂-induced CAR-T group with the hypoxic promoter, and BLANK is a blank control group. The CAR construct modified by the hypoxic promoter increases CAR expression abundance under the hypoxic environment, and the CAR molecule is expressed on the surface of T cells at a higher density.

### Embodiment 4 Detection of effectiveness of CAR-T containing a hypoxic promoter after hypoxia induction

In this embodiment, the CAR-T of the 3H1P promoter, the CAR-T of the 4H1P promoter and the CAR-T of the 5H1P promoter were used for experiments, respectively.

PSCA-positive Hela cells and CEA-positive DLD1-CEA cells modified *in vitro* were respectively used as target cells to verify the effectiveness of CAR-T after the promoter modification. The effector cells (containing CAR-T cells with the CAR expression modified by promoter) were pretreated overnight with hypoxia, and then CAR-T cells were collected and plated in the target cells according to a certain effector-target ratio. The ACEA xCELLigence RTCA MP instrument was used to detect a killing capacity of different CAR-Ts on target cells, and the experimental procedures were carried out according to the instrument instructions. The principle of ACEA xCELLigence RTCA MP is to record the resistance index of the tumor cells attached to the bottom of the well every 15 minutes, and the resistance index is used to determine the proliferation or death of the adherent target cells. The formula result analysis by means of resistance index is: CAR-T cell killing rate = baseline resistance index - real time resistance index.

The experimental results of CAR-T of the 3H1P promoter are shown in Fig. 5a: Compared with the control group, both of CAR-T cells with different constructs containing the hypoxic promoter 3H1P and CAR-T cells with different constructs containing the hypoxic promoter 4H1P can function normally.

The experimental results of CAR-T of the 4H1P promoter are shown in Fig. 5b: Compared with the control group, both of CAR-T cells with different constructs containing the hypoxic promoter 3H1P and CAR-T cells with different constructs containing the hypoxic promoter 4H1P can function normally.

The experimental results of CAR-T of the 5H1P promoter are shown in Fig. 5c1 and 5c2: Fig. 5cl shows the PSCA expression of Hela target cells; Fig. 5c2 shows the killing efficiency of CAR-T in each group on the Hela target cells 24 hours after the addition of CAR-T. Among them, Medium refers to medium only, Medium+ refers to the medium that induces the hypoxic environment, 5H1P-PSCA is CAR-T containing hypoxic promoter 5H1P, 5H1P-PSCA+ is CAR-T containing hypoxic promoter 5H1P under the hypoxic environment, CAG-PSCA is CAR-T without hypoxic promoter, and 5H1P-GFP+ is negative control. Under the hypoxic environment induced by drugs, the killing effect of 5H1P-PSCA+ as claimed in the present disclosure is significantly enhanced, and is greater than that of the CAG-PSCA group without the hypoxic promoter.

Fig. 6a and 6b further verify the regulation of the 3H1P promoter (Fig. 6a) and 4H1P promoter (Fig. 6b) which are the hypoxia-specific promoters modified under the hypoxic and non-hypoxic environments on the killing activity of CAR-T. The results show that the hypoxia-specific promoters can specifically induce CAR-T to kill efficiently under a low effector- target ratio of 1:5 under the hypoxic environment, while the conventional CAR-T has extremely poor killing ability under the hypoxic environment, and the killing percentage value is shown in Table 3 below:

**Table 3 Numerical value of killing percentage**

| Kill percentage | Repeat 1 (%) | Repeat 2 (%) | Repeat 3 (%) |
|---|---|---|---|
| CT | 0.00 | 7.99 | 0.00 |
| 7 | 21.79 | 31.84 | 33.94 |
| 7+ (hypoxia) | 55.81 | 48.68 | 54.70 |
| 8 | 0.00 | 2.59 | 4.32 |
| 8+ (hypoxia) | 37.93 | 35.87 | 36.99 |

The cell supernatant was collected 24 hours after killing, and an IFN-γ and IL-2 secretion ability of a CAR-T cell was detected after stimulation by target cells.

### Embodiment 5 Detection of IFN-γ via Elisa assay

In this embodiment, the CAR-T of the 3H1P promoter, the CAR-T of the 4H1P promoter and the CAR-T of the 5H1P promoter were used for experiments, respectively.

The supernatant collected in Embodiment 4 was used to detect the secretion of IFN-γ via enzyme-linked immunosorbent assay (ELISA) method. BD IFN-γ kit (No. 555142) was used for IFN-γ detection and the experimental procedures were carried out according to the product instructions.

The CAR-T results of the 3H1P promoter are shown in Fig. 7a. Treat represents the factor secretion of CAR-T cells modified by the promoter after killing target cell under the hypoxic environment. UnTreat represents the factor secretion of CAR-T cells modified by the promoter after killing target cell under the non-hypoxic environment. The results show that only when CAR-T cells modified by the promoter are activated under the hypoxic environment can they secrete higher level of IFN-γ.

The CAR-T results of the 4H1P promoter are shown in Fig. 7b. Treat represents the factor secretion of CAR-T cells modified by the promoter after killing target cell under the hypoxic environment. UnTreat represents the factor secretion of CAR-T cells modified by the promoter after killing target cell under the non-hypoxic environment. The results show that only when CAR-T cells modified by the promoter are activated under the hypoxic environment can they secrete higher level of IFN-γ.

The CAR-T results of the 5H1P promoter are shown in Fig. 7c1 and 7c2. 5H1P-PSCA is CAR-T with hypoxic promoter 5H1P, 5H1P-PSCA+ is CAR-T with hypoxic promoter 5H1P under the hypoxic environment, CAG-PSCA is CAR-T without hypoxic promoter, and 5H1P-GFP+ is a negative control. After antigen stimulation under the hypoxic environment, a secretion ability of IFN-γ and IL-2, especially IL-2, for CAR-T cells with hypoxic promoter is much higher than that under the non-hypoxic environment; after antigen stimulation under the non-hypoxic environment, a secretion ability of IFN-γ for CAR-T with hypoxic promoter 5H1P is much lower than that of CAR-T CAG-PSCA without hypoxic promoter. It can be seen that after antigen stimulation under the non-hypoxic environment of normal tissues, an IFN-γ secretion ability of 5H1P-PSCA is much lower than that of those without hypoxic promoter and has higher safety, and after antigen stimulation under the hypoxic environment of tumors, the IFN-γ secretion ability of 5H1P-PSCA is enhanced, indicating that 5H1P plays a role under the hypoxic environment; under the hypoxic environment of tumors, an IL-2 secretion ability of 5H1P-PSCA is equivalent to that of CAG-PSCA, which is much higher than the IL-2 secretion ability of 5H1P-PSCA under the non-hypoxic environment. The 5HRE-CMVmini promoter according to the present disclosure can indeed improve the safety and effectiveness of CAR-T under the hypoxic environment.

### Embodiment 6 Verification of in vivo effectiveness of CAR-T cells with hypoxic promoter

This experiment is verified with 5H1P CD19 CAR-T.

A mouse tumor-bearing model of human lymphoma cells (Raji, CD19 positive) and a mouse tumor-bearing model of human colorectal cancer were established to verify the effect of the addition of the hypoxic promoter on the anti-tumor efficacy of CAR-T cells.

The mice used for the *in vivo* verification were NOD. Cg-PrkdcscidII2rgtmlSug/JicCrl, referred to as NOG mice for short, which were cultivated by Mamoru Ito of Central Institute for Experimental Animals (CIEA), and were the most common strains for CAR-T *in vivo* tumor formation experiments in the world. Raji cells were selected as tumor-forming target cells used in the *in vivo* verification to construct a mouse tumor-bearing model of human lymphoma. Female NOD/SCID mice, aged 6-8 weeks, were selected, and marked with ear tags, and then LoVo or Raji cells (1 × 10⁶ cells/mouse) were subcutaneously injected on the back of the mice. The tumor volume of mice was measured on the 6th day of tumor formation. According to the tumor volume, the mice were randomly divided into two parts for experiment: Control T group, CAR-T modified by the hypoxic promoter (5H1P CD19 CAR-T), and CAR-T group without modification by the promoter (CD19 CAR- T) and CAR-T modified by the hypoxic promoter (5H1P CEA CAR-T) and the control group. The groups of mouse tumor-bearing human lymphoma were intravenously injected with the corresponding CAR-T cells (3*10^6 CAR-T cells/mouse) into the tail of mice in different groups on the 8th day of tumor formation. The Control T group was re-infused with T lymphocytes of a same quantity on the 8th day. The groups of mouse tumor-bearing human colorectal cancer were intravenously injected with 5H1P CEA CAR-T and CONTROL T cells (1*10^6 CAR-T cells/mouse) into the tail of mice in different groups on the 7th day of tumor formation; 5H1P CEA CAR-T and CONTROL T cells (1*10^6 CAR-T cells/mouse) were re-infused again on the 14th day.

The tumor volume of each group of mice was measured once a week, and the experimental results were shown in Fig. 8a and 8b and Fig. 9a and 9c. Fig. 8a shows an experiment of Raji tumor-bearing mice. It can be seen that the *in vivo* effectiveness of the 5H1P group (5H1P CD19 CAR-T) modified with the hypoxia promoter is significantly increased than the unmodified CD19 CAR-T group. In view of the above, the results of in vivo animal experiments show that the CAR-T cells modified with the CAR construct of the 5HRE-CMVmini promoter according to the present disclosure are significantly more effective against the tumor-bearing mice.

Fig. 9a and Fig. 9b show experiments performed by means of a mouse tumor-bearing model of human colorectal cancer. Fig. 9a shows the tumor proliferation curves of the 5H1P group modified by re-infusing the hypoxic promoter and unmodified CAR-T groups. Fig. 9b shows the statistical results of mouse tumor volume after treatment in different modified groups. The results show that the 5H1P group (5H1P CEA CAR-T) modified with the hypoxia promoter has significantly higher efficacy than Control.

The results show that the CAR-T cells modified with the CAR construct of the 5HRE-CMVmini promoter according to the present disclosure are significantly more effective against the tumor-bearing mice.

Finally, it should be noted that the above embodiments are only used to illustrate the technical solutions according to the present disclosure and are not intended to limit. Although the present disclosure has been described in detail with reference to the preferred embodiments, a person of ordinary skill in the art should understand that the technical solution according to the present disclosure can be modified or equivalently replaced without departing from the purpose and scope of the technical solution according to the present disclosure, which should all be covered by the scope of the claims of the present disclosure.

## Claims

1. A promoter, wherein the promoter is hypoxia-regulatable and composed of a Hif1α regulatory element and a mini promoter that are linked to each other, and a quantity of repeats in the Hif1α regulatory element is 3 to 5.

2. The promoter according to claim 1, wherein the quantity of repeats in the Hif1α regulatory element is 3, and a nucleotide sequence of the Hif1α regulatory element is set forth in SEQ ID NO: 1.

3. The promoter according to claim 2, wherein a structure of the Hif1α regulatory element is optimized to obtain an optimized Hif1α regulatory element, and a nucleotide sequence of the Hif1α regulatory element is set forth in SEQ ID NO: 2.

4. The promoter according to any one of claims 2-3, wherein a nucleotide sequence of the promoter is set forth in SEQ ID NO: 3.

5. The promoter according to claim 1, wherein the quantity of repeats in the Hif1α regulatory element is 4, and a nucleotide sequence of the Hif1α regulatory element is set forth in SEQ ID NO: 4.

6. The promoter according to claim 5, wherein a structure of the Hif1α regulatory element is optimized to obtain an optimized Hif1α regulatory element, a nucleotide sequence of the Hif1α regulatory element is set forth in SEQ ID NO: 5.

7. The promoter according to any one of claims 5-6, wherein a nucleotide sequence of the promoter is set forth in SEQ ID NO: 6.

8. The promoter according to claim 1, wherein the quantity of repeats in the Hif1α regulatory element is 5.

9. The promoter according to claim 8, wherein a nucleotide sequence of the promoter is set forth in ID NO: 7.

10. The promoter according to claim 9, wherein the SEQ ID NO: 7 is processed to obtain a promoter having a nucleotide sequence set forth in SEQ ID NO: 8.

11. The promoter according to any one of claims 1-10, wherein the mini promoter is selected from any one of a cell virus promoter, an HSV thymidine kinase promoter, a simian virus 40 promoter, an adenovirus late promoter, and a synthetic promoter.

12. A CAR construct containing the promoter according to any one of claims 1-11.

13. The CAR construct according to claim 12, wherein an antigen recognized by an antigen recognition domain of the CAR construct is one or more of CD19, CD20, CD123, CD22, BCMA, ROR1, mesothelin, PSCA, PSMA, c-Met, GPC-3, Her2, EGFRvIII, GD-2, NY-ESO-1TCR, and MAGEA3TCR.

14. The CAR construct according to claim 13, wherein the antigen recognition region of the CAR construct is ScFv, and an amino acid sequence of the ScFv is set forth in SEQ ID NO: 9 or SEQ ID NO: 10 or SEQ ID NO: 11 or SEQ ID NO: 12 or SEQ ID NO: 13 or a functional variant thereof, or the ScFv contains a heavy chain variable region having an amino acid sequence set forth in SEQ ID NO: 14 and a light chain variable region having an amino acid sequence set forth in SEQ ID NO: 15, or contains a heavy chain variable region having an amino acid sequence set forth in SEQ ID NO: 16 and a light chain variable region having an amino acid sequence set forth in SEQ ID NO: 17, or contains a heavy chain variable region having an amino acid sequence set forth in SEQ ID NO: 18 and a light chain variable region having an amino acid sequence set forth in SEQ ID NO: 19.

15. The CAR construct according to claim 12, wherein an amino acid sequence of a hinge region of the CAR construct is set forth in SEQ ID NO: 20 or SEQ ID NO: 21 or SEQ ID NO: 22 or SEQ ID NO: 23 or SEQ ID NO: 24 or SEQ ID NO: 25 or a functional variant thereof.

16. The CAR construct according to claim 12, wherein an amino acid sequence of a transmembrane region of the CAR construct is set forth in SEQ ID NO: 26 or SEQ ID NO: 27 or a functional variant thereof.

17. The CAR construct according to claim 12, wherein an amino acid sequence of an intracellular costimulatory domain of the CAR construct is set forth in SEQ ID NO: 28 or SEQ ID NO: 29 or SEQ ID NO: 30 or SEQ ID NO: 31 or a functional variant thereof.

18. The CAR construct according to claim 12, wherein an amino acid sequence of an intracellular activation signal of the CAR construct is set forth in SEQ ID NO: 32 or a functional variant thereof.

19. The CAR construct according to claim 12, wherein an amino acid sequence of the CAR construct is set forth in SEQ ID NO: 33 or SEQ ID NO: 34 or SEQ ID NO: 35 or SEQ ID NO: 36 or a functional variant thereof.

20. An isolated nucleic acid molecule, encoding the CAR construct according to any one of claims 19.

21. A CAR expression vector containing the promoter according to any one of claims 1-11, wherein the nucleotide sequence of the hypoxia promoter is set forth in SEQ ID NO: 1 or SEQ ID NO: 2 or SEQ ID NO: 3 or SEQ ID NO: 4 or SEQ ID NO: 5 or SEQ ID NO: 6 or SEQ ID NO: 7 or SEQ ID NO: 8; and the expression vector is any one of a lentivirus expression vector, a retrovirus expression vector, an adenovirus expression vector, an adeno-associated virus expression vector, a DNA vector, an RNA vector and a plasmid.

22. The CAR expression vector according to claim 21, wherein a nucleotide sequence of the vector is set forth in SEQ ID NO: 37 or SEQ ID NO: 38 or SEQ ID NO: 39 or SEQ ID NO: 40 or SEQ ID NO: 41 or SEQ ID NO:42 or SEQ ID NO:43 or SEQ ID NO:44.

23. The CAR expression vector according to claim 21, wherein when the quantity of repeats in the Hif1α regulatory element is 5, the expression vector is pBKL1-5H1P-CAR/anti-PSCA-oPRE, which contains the CAR construct having a nucleotide sequence set forth in SEQ ID NO: 45; the vector is pBKL1-5H1P-CAR/anti-CD19-oPRE, which contains the CAR construct having a nucleotide sequence set forth in SEQ ID NO: 46; and the vector is pBKL1-5H1P-CAR/anti-CEA-oPRE, which contains the CAR construct having a nucleotide sequence set forth in SEQ ID NO: 47.

24. A CAR-T cell containing the expression vector according to any one of claims 21-23.

25. A method for improving a killing capacity of a CAR-T cell under a hypoxic environment, comprising constructing the CAR expression vector according to any one of claims 21-23 and infecting T lymphocytes, and acting on target cells.

26. A method for improving an ability of a CAR-T cell to secrete IFN-γ and/or IL-2 factors under a hypoxic environment, comprising constructing a CAR construct containing a hypoxia-regulatable promoter, wherein the hypoxia-regulatable promoter is composed of a Hif1α regulatory element and a mini promoter that are linked to each other, and a quantity of repeats in the Hif1α regulatory element is 3 to 5; and the mini promoter is selected from a cell virus promoter, a HSV thymidine kinase promoter, a simian virus 40 promoter, an adenovirus late promoter, or a synthetic promoter.

27. The use of the promoter according to claim 1 or the CAR construct according to claim 12 or the nucleic acid molecule according to claim 20 or the expression vector according to claim 21 or the CAR-T cell according to claim 24 in preparation of tumor drugs.

28. The use according to claim 27, wherein the tumor comprises acute lymphoid leukemia, chronic lymphocytic leukemia, chronic myelogenous leukemia, non-Hodgkin's lymphoma, Hodgkin's lymphoma, prostate cancer, colorectal cancer, breast cancer, ovarian cancer, cervical cancer, pancreatic cancer, lung cancer, kidney cancer, liver cancer, brain cancer and skin cancer; and the tumor highly expresses one or more of CD19, CD20, CD123, CD22, BCMA, ROR1, CEA, mesothelin, PSCA, PSMA, c-Met, GPC-3, Her2, EGFRvIII, GD-2, NY-ES0-1TCR, and MAGE A3TCR.
